# EUROPÄISCHE PATENTSCHRIFT

(11) **EP 0 811 007 B1**
(45) Veröffentlichungstag und Bekanntmachung des Hinweises auf die Patenterteilung: **23.11.2005**
(21) Anmeldenummer: 96902274.8
(22) Anmeldetag: 14.02.1996
(51) Int. Cl.: C07D 409/10, C07D 413/10, C07D 417/10, A01N 43/56

(54) **PYRAZOL-4-YL-BENZOYLDERIVATE UND IHRE VERWENDUNG ALS HERBIZIDE**
PYRAZOL-4-YL-BENZOYL DERIVATIVES AND THEIR USE AS HERBICIDES
DERIVES DE PYRAZOL-4-YL-BENZOYLE ET LEUR UTILISATION COMME HERBICIDES

(30) Priorität: 24.02.1995 DE 19506572
(43) Veröffentlichungstag der Anmeldung: 10.12.1997
(73) Patentinhaber: BASF Aktiengesellschaft, 67056 Ludwigshafen (DE)
(72) Erfinder: VON DEYN, Wolfgang, D-67434 Neustadt (DE); HILL, Regina, Luise, D-67346 Speyer (DE); KARDORFF, Uwe, D-68159 Mannheim (DE); ENGEL, Stefan, D-65510 Idstein (DE); OTTEN, Martina, D-67069 Ludwigshafen (DE); VOSSEN, Marcus, D-68199 Mannheim (DE); PLATH, Peter, D-67227 Frankenthal (DE); RANG, Harald, D-67122 Altrip (DE); HARREUS, Albrecht, D-67063 Ludwigshafen (DE); KÖNIG, Hartmann, D-69115 Heidelberg (DE); WALTER, Helmut, D-67283 Obrigheim (DE); WESTPHALEN, Karl-Otto, D-67346 Speyer (DE); MISSLITZ, Ulf, D-67433 Neustadt (DE)
(86) Internationale Anmeldenummer: PCT/EP1996/000635
(87) Internationale Veröffentlichungsnummer: WO 1996/026206

(56) Entgegenhaltungen:
- EP-A- 0 282 944
- EP-A- 0 410 552
- F.D. KING: "Medicinal Chemistry: Principles and Practice" 1994 , THE ROYAL SOCIETY OF CHEMISTRY , CAMBRIDGE, GB XP002000848 siehe Seite 208; Tabelle 2, 3. Eintrag: "CARBOXYLIC ESTER GROUP"

## Beschreibung

Die vorliegende Erfindung betrifft neue Pyrazolyl-benzoylderivate mit herbizider Wirkung, Verfahren zur Herstellung der Pyrazolyl-benzoylderivate, Mittel welche diese enthalten sowie die Verwendung dieser Derivate oder sie enthaltender Mittel zur Unkrautbekämpfung.

Aus der Literatur sind herbizidwirksame Pyrazolyl-benzoylderivate bekannt, beispielsweise aus EP 352543.

Weiterhin beschreibt EP 282944 4-Benzoyl-5-hydroxypyrazole, welche in meta-Position des Benzoyl-Restes beispielsweise eine Estergruppe tragen.

Die herbiziden Eigenschaften der bekannten Verbindungen sowie die Verträglichkeit gegenüber Kulturpflanzen können jedoch nur bedingt befriedigen.

Die Aufgabe bestand darin neue Pyrazolyl-benzoylderivate mit verbesserten Eigenschaften zu finden.

Es wurden nun neue Pyrazolyl-benzoylderivate der Formel I gefunden 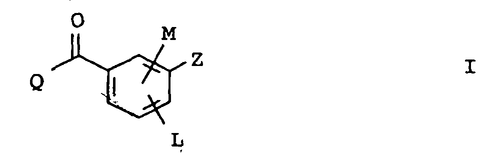 in der die Substituenten folgende Bedeutung haben:
- L,M: Wasserstoff, C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können, Halogen, Cyano, Nitro, eine Gruppe -(Y)ₙ-S(O)ₘR⁷ oder eine Gruppe -(Y)ₙ-CO-R⁸
- Z: ein 5- oder 6-gliedriger heterocyclischer, gesättigter oder ungesättigter Rest, enthaltend ein bis drei Heteroatome, ausgewählt aus der Gruppe Sauerstoff, Schwefel oder Stickstoff, der gegebenenfalls durch Halogen, Cyano, Nitro, eine Gruppe -CO-R⁸, C₁-C₄-Alkyl, C₁-C₄-Halogenalkyl, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, gegebenenfalls durch Halogen, Cyano, Nitro, C₁-C₄-Alkyl oder C₁-C₄-Halogenalkyl substituiertes Phenyl oder eine Oxogruppe, die gegebenenfalls auch in der tautomeren Form als Hydroxygruppe vorliegen kann, substituiert ist oder der mit einem ankondensierten, gegebenenfalls durch Halogen, Cyano, Nitro, C₁-C₄-Alkyl oder C₁-C₄-Halogenalkyl substituierten Phenylring, einem ankondensierten Carbocyclus oder einem ankondensierten, gegebenenfalls durch Halogen, Cyano, Nitro, C₁-C₄-Alkyl, Di-C₁-C₄-Alkylamino, C₁-C₄-Alkoxy, C₁-C₄-Halogenalkoxy, oder C₁-C₄-Halogenalkyl substituierten zweiten Heterocyclus ein bicyclisches System bildet.
- Y: O, NR⁹
- n: null oder eins
- m: null, eins oder zwei
- R⁷: C₁-C₄-Alkyl, C₁-C₄-Halogenalkyl oder NR⁹R¹⁰
- R⁸: C₁-C₄-Alkyl, C₁-C₄-Halogenalkyl, C₁-C₄-Alkoxy, oder NR⁹R¹⁰
- R⁹: Wasserstoff oder C₁-C₄-Alkyl
- R¹⁰: C₁-C₄-Alkyl
- Q: ein in Stellung 4 verknüpfter Pyrazolring der Formel II 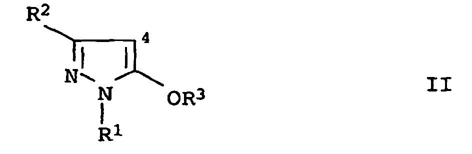
in welcher
- R¹: C₁-C₄-Alkyl
- R²: Wasserstoff, C₁-C₄-Alkyl oder C₁-C₄-Halogenalkyl und
- R³: Wasserstoff, C₁-C₄-Alkylsulfonyl, Phenylsulfonyl oder Alkylphenylsulfonyl bedeuten,
sowie landwirtschaftlich übliche Salze der Verbindungen I.

Verbindungen der Formel I erhält man dadurch, daß man 5-Hydroxypyrazole der Formel IIa mit einem Benzoylderivat der Formel III CT = Cl) acyliert und den gebildeten Pyrazolester zu den Verbindungen der Formel Ic umlagert.

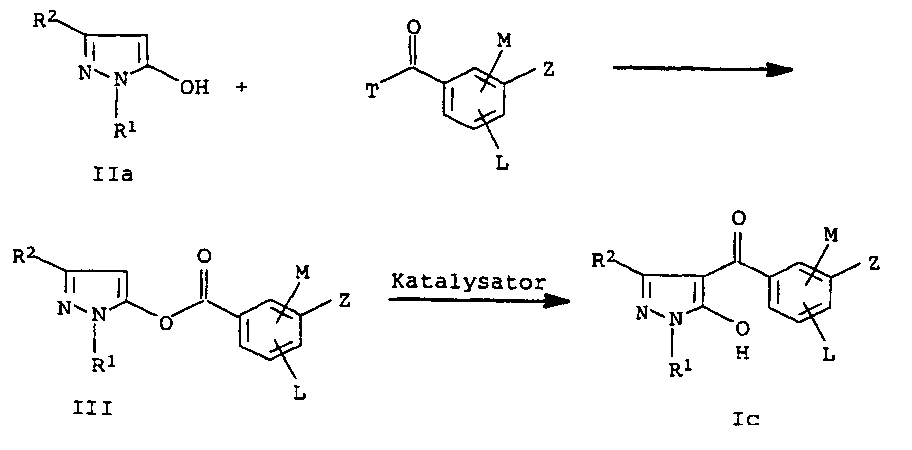

In den oben genannten Formeln haben T = Halogen und L, M und Z die eingangs angegebene Bedeutung.

Der erste Schritt der Reaktionsabfolge, die Acylierung, erfolgt in allgemein bekannter Weise, z. B. durch Zugabe eines Benzoylderivates der Formel III (T = Cl) zur Lösung oder Suspension eines 5-Hydroxypyrazols IIa in Gegenwart einer Hilfsbase. Die Reaktanden und die Hilfsbase werden dabei zweckmäßigerweise in etwa äquimolaren Mengen eingesetzt. Ein geringer Überschuß der Hilfsbase, z.B. 1,2 bis 1,5 Moläquivalente, bezogen auf II, kann u.U. von Vorteil sein.

Als Hilfsbase eignen sich z.B. tertiäre Alkylamine, Pyridin oder Alkalicarbonate, während als Lösungsmittel Methylenchlorid, Diethylether, Toluol oder Essigsäureethylester verwendet werden können. Während der Zugabe des Säurechlorids wird die Reaktionsmischung vorteilhaft auf 0 -10°C gekühlt, danach wird bei höherer Temperatur, z.B. bei einer Temperatur von 25 - 50°C gerührt, bis die Umsetzung beendet ist.

Die Aufarbeitung erfolgt in üblicher Weise, z.B. wird das Reaktionsgemisch in Wasser gegossen und mit Methylenchlorid extrahiert. Nach Trocknen der organischen Phase und Entfernung des Lösungsmittels kann der rohe 5-Hydroxypyrazolester ohne weitere Reinigung zur Umlagerung eingesetzt werden. Herstellungsbeispiele für Benzoesäureester von 5-Hydroxy-pyrazolen findet man z. B. in EP-A-282 944 oder US 4,643,757.

Die Umlagerung der 5-Hydroxypyrazolester zu den Verbindungen der Formel I.c erfolgt zweckmäßigerweise bei Temperaturen von 20°C bis 40°C in einem Lösungsmittel und in Gegenwart einer Hilfsbase sowie mit Hilfe einer Cyanoverbindung als Katalysator. Als Lösungsmittel können z.B. Acetonitril, Methylenchlorid, 1,2-Dichlorethan, Essigsäureethylester oder Toluol verwendet werden. Bevorzugtes Lösungsmittel ist Acetonitril. Als Hilfsbase eignen sich tertiäre Alkylamine, Pyridin oder Alkalicarbonate, die in äquimolarer Menge oder bis zu vierfachem Überschuß eingesetzt werden. Bevorzugte Hilfsbase ist Triethylamin in doppelter Menge. Als Katalysator eignen sich Cyanidverbindungen, wie Kaliumcyanid oder Aceton-cyanhydrin, z.B. in einer Menge von 1 bis 50, insbesondere 5-20 Molprozent, bezogen auf den 5-Hydroxy-pyrazolester. Bevorzugt setzt man Acetoncyanhydrin z.B. in Mengen von 10 Molprozent zu.

Beispiele zur Umlagerung von Benzoesäureestern von 5-Hydroxypyrazolen findet man z. B. in EP-A 282 944 oder US 4,643,757, jedoch wird dort lediglich Kaliumcarbonat oder Natriumcarbonat in Dioxan als Katalysator verwendet. Die Verwendung von Kaliumcyanid oder Acetoncyanhydrin ist zwar im Zusammenhang mit der analogen Umlagerung von Enolestern von Cyclohexan-1,3-dionen bekannt (US 4,695,673), jedoch sind aus der Literatur keine Beispiele bekannt, daß sich Cyanidverbindungen besonders gut zur Fries-Umlagerung von O-Acylderivaten des 5-Hydroxypyrazols eignen.

Die Aufarbeitung erfolgt in üblicher Weise, z.B. wird das Reaktionsgemisch mit verdünnten Mineralsäuren wie 5 % Salzsäure oder Schwefelsäure angesäuert und extrahiert z.B. mit Methylenchlorid oder Essigsäureethylester. Zur Reinigung wird der Extrakt mit kalter 5 - 10 % Alkalicarbonatlösung extrahiert, wobei das Endprodukt in die wäßrige Phase übergeht. Durch Ansäuern der wäßrigen Lösung wird das Produkt der Formel Ic ausgefällt, oder erneut mit Methylenchlorid extrahiert, getrocknet und anschließend vom Lösungsmittel befreit.

Die als Ausgangsmaterial verwendeten 5-Hydroxypyrazole der Formel II sind bekannt und können nach an sich bekannten Verfahren hergestellt werden (vgl. EP- A 240 001 und J. Prakt. Chem. 315, 382 (1973)). 1,3-Dimethyl-5-hydroxypyrazol ist eine käufliche Verbindung.

Benzoesäurederivate der Formel III lassen sich folgendermaßen herstellen:

Benzoylhalogenide wie beispielsweise Benzoylchloride der Formel III (T = Cl) werden in an sich bekannter Weise durch Umsetzung der Benzoesäuren der Formel III (T = OH) mit Thionylchlorid hergestellt.

Die Benzoesäuren der Formel III (T = OH) können in bekannter Weise durch saure oder basische Hydrolyse aus den entsprechenden Estern der Formel III (T = C₁-C₄-Alkoxy) hergestellt werden.

Die Zwischenprodukte der Formel III lassen sich z.B. gemäß Schema 2 und 3 auf den im folgenden beschriebenen wegen darstellen. 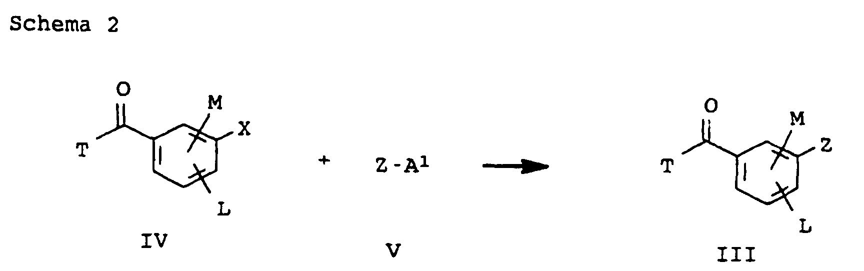
- T: C₁-C₄-Alkoxy,
- X: Cl, Br, J, -OS(O)₂CF₃, -OS(O)₂F
- A¹: Sn (C₁-C₄-Alkyl)₃, B (OH)₂, ZnHal, wobei Hal für Cl oder Br steht
- L, M, Z: wie oben definiert.

Danach lassen sich die Arylhalogenverbindungen oder Arylsulfonate IV in an sich bekannter Weise mit Heteroarylstannaten (Stille-Kupplungen), Heteroaryl-Borverbindungen (Suzuki-Kupplungen) oder Heteroaryl-Zinkverbindungen (Negishi-Reaktion) V (vgl. z.B. Synthesis 1987, 51-53, Synthesis 1992, 413) in Gegenwart eines Palladium- oder Nickel-Übergangsmetallkatalysators und gegebenenfalls einer Base zu den neuen Verbindungen der allgemeinen Formel III umsetzen.

Die Benzoesäurederivate der Formel III können auch erhalten werden, indem man entsprechende brom- oder iodsubstituierte Verbindungen der Formel VI 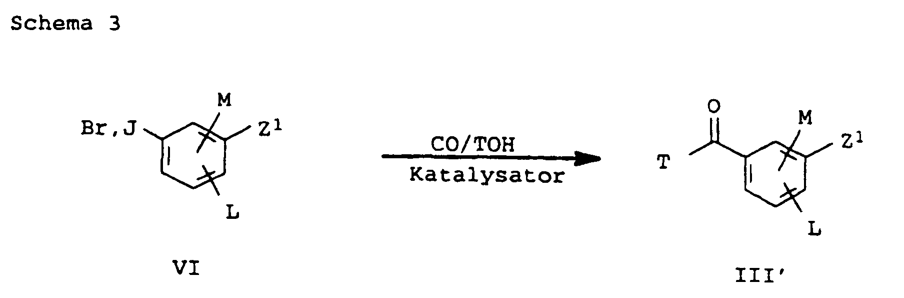
- Z¹: Z oder CN
- T: OH, C₁-C₄-Alkoxy
in der L und M die obengenannte Bedeutung haben, in Gegenwart eines Palladium-, Nickel-, Cobalt- oder Rhodium-Übergangsmetallkatalysators und einer Base mit Kohlenmonoxid und Wasser unter erhöhtem Druck umsetzt.

Bevorzugt im Rahmen der vorliegenden Erfindung sind Benzoylderivate der Formel IIIa 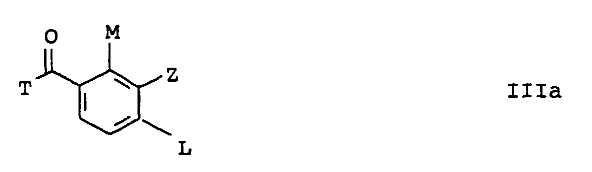 in der T, L, M und Z die folgende Bedeutung haben:
- T: Chlor, OH oder C₁-C₄-Alkoxy
- L: C₁-C₆-Alkyl, C₂-C₆-Alkenyl, C₂-C₆-Alkinyl, C₁-C₄-Alkoxy, C₁-C₄-Alkylthio, C₁-C₄-Halogenalkyl, C₁-C₄-Halogenalkoxy, C₁-C₄-Halogenalkylthio, C₁-C₄-Alkylsuflonyl, Halogen, Nitro oder Cyano
- M: C₁-C₆-Alkyl, C₂-C₆-Alkenyl, C₂-C₆-Alkinyl, C₁-C₄-Alkoxy, C₁-C₄-Alkylthio, C₁-C₄-Halogenalkyl, C₁-C₄-Halogenalkoxy, C₁-C₄-Halogenalkylthio, C₁-C₄-Alkylsulfonyl, Halogen, Nitro oder Cyano
- Z: wie oben angegeben.

Bevorzugt sind auch Benzoylderivate der Formel IIIb 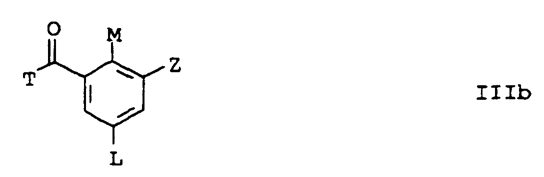 in der T,L,M und Z die folgende Bedeutung haben:
- T: Chlor, OH oder C₁-C₄-Alkoxy
- L,M: C₁-C₆-Alkyl, C₂-C₆-Alkenyl, C₂-C₆-Alkinyl, C₁-C₄-Alkoxy, C₁-C₄-Alkylthio, C₁-C₄-Halogenalkyl, C₁-C₄-Halogenalkoxy, C₁-C₄-Halogenalkylthio, C₁-C₄-Alkylsulfonyl, Halogen, Nitro oder Cyano
- Z: wie oben angegeben.

Die Katalysatoren Nickel, Cobalt, Rhodium und insbesondere Palladium können metallisch oder in Form üblicher Salze wie in Form von Halogenverbindungen, z.B. PdCl₂, RhCl₃-H₂O, Acetaten, z.B. Pd(OAc)₂, Cyaniden usw. in den bekannten Wertigkeitsstufen vorliegen. Ferner können Metallkomplexe mit tertiären Phosphinen, Metallalkylcarbonyle, Metallcarbonyle, z.B. CO₂(CO)₈, Ni(CO)₄, Metallcarbonyl-Komplexe mit tertiären Phosphinen, z.B. (PPh₃)₂Ni(CO)₂, oder mit tertiären Phosphinen komplexierte Übergangsmetallsalze vorliegen. Die letztgenannte Ausführungsform ist insbesondere im Fall von Palladium als Katalysator bevorzugt. Dabei ist die Art der Phosphinliganden breit variabel. Beispielsweise lassen sie sich durch folgende Formeln wiedergeben: 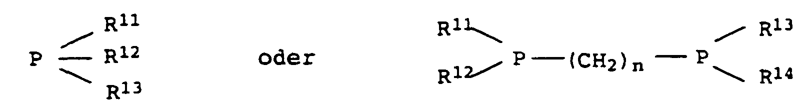 wobei n die Zahlen 1, 2, 3 oder 4 bedeutet und die Reste R¹¹ bis R¹⁴ für niedermolekulares Alkyl, z.B. C₁-C₆-Alkyl, Aryl, C₁-C₄-Alkylaryl, z.B. Benzyl, Phenethyl oder Aryloxy stehen. Aryl ist z.B. Naphthyl, Anthryl und vorzugsweise gegebenenfalls substituiertes Phenyl, wobei man hinsichtlich der Substituenten nur auf deren Inertheit gegenüber der Carboxylierungsreaktion zu achten hat, ansonsten können sie breit variiert werden und umfassen alle inerten C-organischen Reste wie C₁-C₆-Alkylreste, z.B. Methyl, Carboxylreste wie COOH, COOM (M ist z.B. ein Alkali-, Erdalkalimetall oder Ammoniumsalz), oder C-organische Reste über Sauerstoff gebunden wie C₁-C₆-Alkoxyreste.

Die Herstellung der Phosphinkomplexe kann in an sich bekannter Weise, z.B. wie in den eingangs genannten Dokumenten beschrieben, erfolgen. Beispielsweise geht man von üblichen kommerziell erwerblichen Metallsalzen wie PdCl₂ oder Pd(OCOCH₃)₂ aus und fügt das Phosphin z.B. P(C₆H₅)₃, P(n-C₄H₉)₃, PCH₃(C₆H₅)₂, 1,2-Bis(diphenylphosphino)ethan hinzu.

Die Menge an Phosphin, bezogen auf das Übergangsmetall, beträgt üblicherweise 0 bis 20, insbesondere 0,1 bis 10 Moläquivalente, besonders bevorzugt 1 bis 5 Moläquivalente.

Die Menge an Übergangsmetall ist nicht kritisch. Natürlich wird man aus Kostengründen eher eine geringe Menge, z.B. von 0,1 bis 10 Mol.-%, insbesondere 1 bis 5 Mol.-%, bezogen auf den Ausgangsstoff VI verwenden.

Zur Herstellung der Benzoesäuren III (T = OH) führt man die Umsetzung mit Kohlenmonoxid und mindestens äquimolaren Mengen an Wasser, bezogen auf die Ausgangsstoffe VI durch. Der Reaktionspartner Wasser kann gleichzeitig auch als Lösungsmittel dienen, d.h. die maximale Menge ist nicht kritisch.

Es kann aber auch je nach Art der Ausgangsstoffe und der verwendeten Katalysatoren von Vorteil sein, anstelle des Reaktionspartners ein anderes inertes Lösungsmittel oder die für die Carboxylierung verwendete Base als Lösungsmittel zu verwenden.

Als inerte Lösungsmittel kommen für Carboxylierungsreaktionen übliche Lösungsmittel wie Kohlenwasserstoffe, z.B. Toluol, Xylol, Hexan, Pentan, Cyclohexan, Ether z.B. Methyl-tert.butylether, Tetrahydrofuran, Dioxan, Dimethoxyethan, substituierte Amide wie Dimethylformamid, persubstituierte Harnstoffe wie Tetra-C₁-C₄-alkylharnstoffe oder Nitrile wie Benzonitril oder Acetonitril in Betracht.

In einer bevorzugten Ausführungsform des Verfahrens verwendet man einen der Reaktionspartner, insbesondere die Base, im Überschuß, so daß kein zusätzliches Lösungsmittel erforderlich ist.

Für das Verfahren geeignete Basen sind alle inerten Basen, die den bei der Umsetzung freiwerdenden Jodwasserstoff bzw. Bromwasserstoff zu binden vermögen. Beispielsweise sind hier tertiäre Amine wie tert.-Alkylamine, z.B. Trialkylamine wie Triethylamin, cyclische Amine wie N-Methylpiperidin oder N,N'-Dimethylpiperazin, Pyridin, Alkali- oder -hydrogencarbonate, oder tetraalkylsubstituierte Harnstoffderivate wie Tetra-C₁-C₄-alkylharnstoff, z.B. Tetramethylharnstoff, zu nennen.

Die Menge an Base ist nicht kritisch, üblicherweise werden 1 bis 10, insbesondere 1 bis 5 Mol verwendet. Bei gleichzeitiger Verwendung der Base als Lösungsmittel, wird die Menge in der Regel so bemessen, daß die Reaktionspartner gelöst sind, wobei man aus Praktikabilitätsgründen unnötig hohe Überschüsse vermeidet, um Kosten zu sparen, kleine Reaktionsgefäße einsetzen zu können und den Reaktionspartnern maximalen Kontakt zu gewährleisten.

Während der Umsetzung wird der Kohlenmonoxiddruck so eingestellt, daß immer ein Überschuß an CO, bezogen auf VI vorliegt. vorzugsweise liegt der Kohlenmonoxiddruck bei Raumtemperatur bei 1 bis 250 bar, insbesondere 5 bis 150 bar CO.

Die Carbonylierung wird in der Regel bei Temperaturen von 20 bis 250°C, insbesondere bei 30 bis 150°C kontinuierlich oder diskontinuierlich durchgeführt. Bei diskontinuierlichem Betrieb wird zweckmäßigerweise zur Aufrechterhaltung eines konstanten Druckes kontinuierlich Kohlenmonoxid auf das Umsetzungsgemisch aufgepreßt.

Die als Ausgangsverbindungen benutzten Arylhalogenverbindungen VI sind bekannt oder können leicht durch geeignete Kombination bekannter Synthesen hergestellt werden.

Beispielsweise können die Halogenverbindungen VI durch Sandmeyer-Reaktion aus entsprechenden Anilinen erhalten werden, die ihrerseits durch Reduktion von geeigneten Nitroverbindungen (vgl. z.B. für VI mit Z¹ = CN: Liebigs Ann. Chem. 1980, 768-778) synthetisiert werden. Die Arylbromide VI können außerdem durch direkte Bromierung geeigneter Ausgangsverbindungen erhalten werden [vgl. z.B. Monatsh. Chem. 99, 815-822 (1968)]. 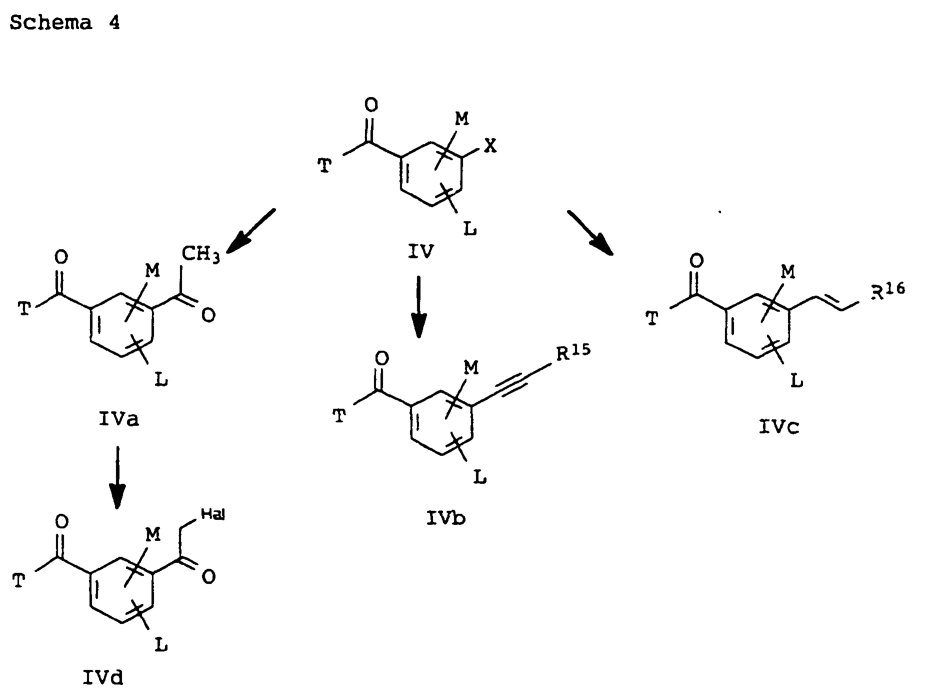
- T: C₁-C₄-Alkoxy
- X: Cl, Br, J, -OS (O)₂CF₃, -OS (O)₂F
- L,M,Z: wie oben definiert
- R¹⁵: Wasserstoff, C₁-C₄-Alkyl, C₁-C₄-Halogenalkyl, C₃-C₈-Cycloalkyl, ggf. subst. Phenyl oder Trimethylsilyl,
- R¹⁶: Wasserstoff, C₁-C₄-Halogenalkyl, C₃-C₈-Cycloalkyl oder ggf. subst. Phenyl.

Ausgehend von den Arylhalogenverbindungen oder Arylsulfonaten IV lassen sich in Gegenwart eines Palladium- oder Nickel-Übergangsmetallkatalysators und gegebenenfalls einer Base Arylmethylketone IVa nach literaturbekannten Verfahren durch Umsetzung mit Vinylalkylethern und anschließende Hydrolyse herstellen [vgl. z.B. Tetrahedron Lett. 32, 1753-1756 (1991)].

Die ethinylierten Aromaten IVb können in an sich bekannter Weise durch Umsetzung von Arylhalogenverbindungen oder Arylsulfonaten IV mit substituierten Acetylenen in Gegenwart eines Palladium- oder Nickel-Übergangsmetallkatalysators hergestellt werden (z.B. Heterocycles, 24, 31-32 (1986)). Derivate IVb mit R¹⁵ = H erhält man zweckmäßigerweise aus den Silylverbindungen IVb, R¹⁵ = -Si(CH₃)₃ [J.Org.Chem. 46, 2280-2286 (1981)].

Durch Heck-Reaktion von Arylhalogenverbindungen oder Arylsulfonaten IV mit Olefinen in Gegenwart eines Palladiumkatalysators werden die Arylalkene IVc erhalten (vgl. z.B. Heck, Palladium Reagents in Organic Synthesis, Academic Press, London 1985 bzw. Synthesis 1993, 735-762).

Die als Ausgangsverbindungen benutzten Benzoylderivate IV sind bekannt [ vgl. z.B.Coll. Czech. Chem. Commn. 40, 3009-3019 (1975)] oder können leicht durch geeignete Kombination bekannter Synthesen hergestellt werden.

Beispielsweise können die Sulfonate IV (X = -OS(O)₂CF₃, -OS(O)₂F) aus den entsprechenden Phenolen, die ihrerseits bekannt sind (vgl. z.B. EP 195247) oder nach bekannten Methoden hergestellt werden können, erhalten werden (vgl. z.B. Synthesis 1993, 735-762).

Die Halogenverbindungen IV (X = Cl, Br oder I) können beispielsweise durch Sandmeyer-Reaktion aus entsprechenden Anilinen erhalten werden. 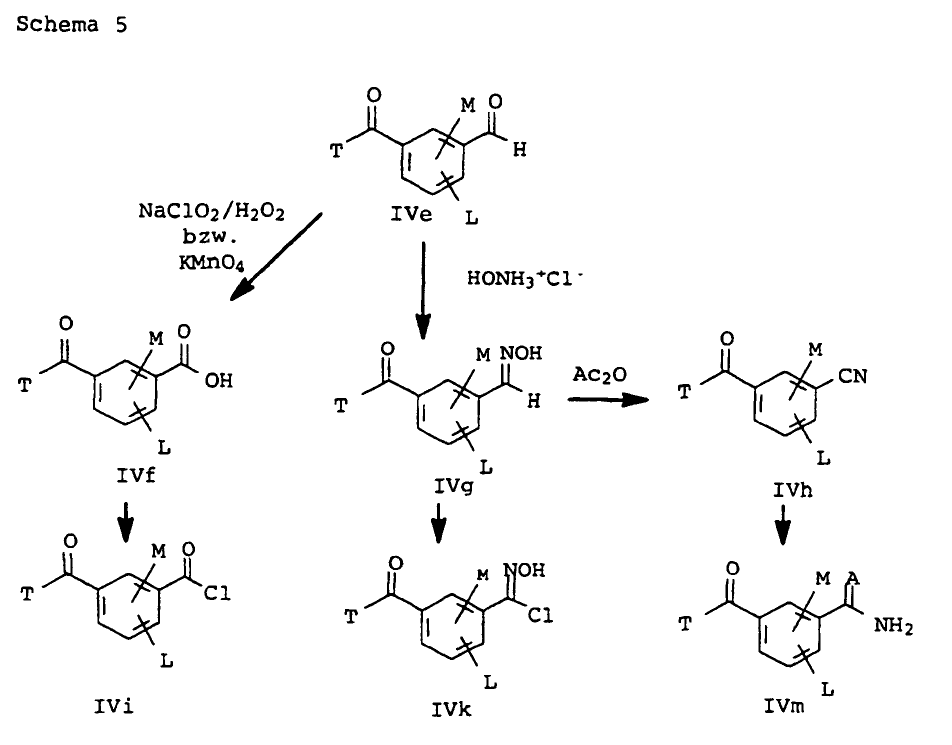
- A: S, NH oder NOH
- T: ist C₁-C₄-Alkoxy und L, M wie oben definiert.

Isophthalsäurederivate IVf können aus den Aldehyden IVe nach bekannten Verfahren hergestellt werden [ s. J. March Advanced Organic Chemistry 3. Aufl., S. 629ff, Wiley-Interscience Publication (1985)].

Die Oxime IVg erhält man vorteilhaft dadurch, daß man in an sich bekannter Weise Aldehyde IVe mit Hydroxylamin umsetzt [ s. J. March Advanced Organic Chemistry 3. Aufl., S. 805-806, wiley-Interscience Publication (1985)].

Die Umwandlung der Oxime IVg in Nitrile IVh kann ebenfalls nach an sich bekannten Verfahren erfolgen [s. J. March Advanced Organic Chemistry 3. Aufl., S. 931-932, Wiley-Interscience Publication (1985)].

Die als Ausgangsverbindungen benötigten Aldehyde IVe sind bekannt oder nach bekannten Methoden herstellbar. Beispielsweise können sie gemäß Schema 6 aus den Methylverbindungen VII synthetisiert werden.

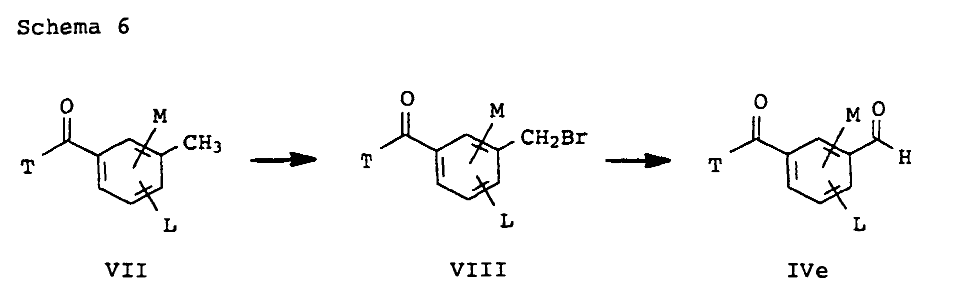

Die Reste T, M und L haben die unter Schema 5 genannte Bedeutung. Die Methylverbindungen VII können nach allgemein bekannten Methoden, beispielsweise mit N-Bromsuccinimid oder 1,3-Dibrom-5,5-dimethylhydantoin, zu den Benzylbromiden VIII umgesetzt werden. Die Umsetzung von Benzylbromiden zu Benzaldehyden IVe ist ebenfalls literaturbekannt [vgl. Synth. Commun. 22 1967-1971 (1992)].

Die Vorprodukte IVa bis IVh eignen sich zum Aufbau heterocyclischer Zwischenprodukte III.

Beispielsweise können aus den Acetophenonen IVa über die halogenierte Zwischenstufe Ivd 5-Oxazolyl-[vgl. z.B. J. Heterocyclic Chem., 28, 17-28 (1991)] oder 4-Thiazolyl-derivate [vgl. z.B. Metzger, Thiazoles in: The Chemistry of heterocyclic compounds, Vol.34 S. 175ff (1976)] erhalten werden.

Die Acetylene IVb bzw. die Alkene IVc eignen sich zum Aufbau von 4-Isoxazolyl-, 5-Isoxazolyl-, 4,5-Dihydroisoxazol-4-yl-, 4,5-Dihydroisoxazol-5-yl-derivaten [vgl. z.B. Houben-Weyl, Methoden der organischen Chemie, 4. Aufl., Bd. X/3, S. 843ff (1965)].

Aus den Benzoesäuren IVf bzw. den daraus nach Standardverfahren erhältlichen Säurechloriden IVi können beispielsweise nach literaturbekannten Verfahren 2-Oxazolyl-, 1,2,4-Oxadiazol-5-yl-, 1,3,4-Oxadiazol-2-yl-derivate [ vgl. z.B. J. Heterocyclic Chem., 28, 17-28 (1991)] oder 2-Pyrrolyl-derivate [ vgl. z.B. Heterocycles 26, 3141-3151 (1987)] hergestellt werden.

1,2,4-Triazol-3-yl-derivate sind aus Benzonitrilen IVh nach bekannten Methoden [ vgl. z.B. J. Chem. Soc. 3461-3464 (1954)] herzustellen.

Aus den Benzoesäuren IVf bzw. den daraus nach Standardverfahren erhältlichen Säurechloriden IVi können beispielsweise nach literaturbekannten Verfahren 2-Oxazolyl-, 1,2,4-Oxadiazol-5-yl-, 1,3,4-Oxadiazol-2-yl-derivate [ vgl. z.B. J. Heterocyclic Chem., 28, 17-28 (1991)] oder 2-Pyrrolyl-derivate [ vgl. z.B. Heterocycles 26, 3141-3151 (1987)] hergestellt werden.

1,2,4-Triazol-3-yl-derivate sind aus Benzonitrilen IVh nach bekannten Methoden [ vgl. z.B. J. Chem. Soc. 3461-3464 (1954)] herzustellen.

Die Benzonitrile IVh können über die Zwischenstufe der Thioamide, Amidoxime oder Amidine IVm in 1,2,4-Oxadiazol-3-yl- [ vgl. z.B. J. Heterocyclic Chem., 28, 17-28 (1991)] 2-Thiazolyl-, 4,5-Dihydro-thiazol-2-yl- oder 5,6-Dihydro-4-H-1,3-thiazin-2-yl-derivate [vgl. z.B. Houben-Weyl, Methoden der organischen Chemie, 4. Aufl., Bd. E5, S. 1268ff (1985)] umgewandelt werden. Aus den Thioamiden IVm (A=S) sind nach literaturbekannten Verfahren auch 1,2,4-Thiadiazol-5-yl-derivate [vgl. z.B. J.Org.Chem. 45 3750-3753 (1980)] oder 1,3,4-Thiadiazol-2-yl-derivate [ vgl. z.B. J. Chem.Soc., Perkin Trans. I 1987-1991 (1982)] erhältlich.

Die Umwandlung von Oximen IVg in 3-Isoxazolyl-derivate kann in an sich bekannter weise über die Zwischenstufe der Hydroxamsäurechloride IVk erfolgen [vgl. z.B. Houben-Weyl, Methoden der organischen Chemie, 4. Aufl., Bd. X/3, S. 843ff (1965)].

Im Hinblick auf die bestimmungsgemäße Verwendung der Benzoylderivate der allgemeinen Formel I kommen als Substituenten folgende Reste in Betracht:
L,M wasserstoff,
C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl. 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 1,1,2-Trimethylpropyl, 1,2,2-Trimethylpropyl, 1-Ethyl-1-methylpropyl oder 1-Ethyl-2-methyl-propyl,
insbesondere Methyl, Ethyl, 1-Methylethyl, 1-Methylpropyl, 2-Methylpropyl, 1,1-Dimethylethyl und 1,1-Dimethylpropyl;
C₂-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 3-Methyl-2-butenyl, 1-Methyl-2-butenyl, 2-Methyl-2-butenyl, 1-Methyl-3-butenyl, 2-Methyl-4-butenyl, 3-Methyl-3 butenyl, 1,1-Dimethyl-2-propenyl,1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3 pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3 butenyl, 2-Ethyl-2-butenyl, 2-Ethyl-3-butenyl, 1,1,2-Trimethyl-2-propenyl, 1-Ethyl-1-methyl-2-propenyl und Ethyl-2-methyl-2-propenyl,
insbesondere 1-Methyl-2-propenyl, 1-Methyl-2-butenyl, 1,1-Dimethyl-2-propenyl und 1,1-Dimethyl-2-butenyl;
C₂-C₆-Alkinyl wie Propargyl, 2-Butinyl, 3-Butenyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2 propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
C₁-C₄-Alkoxy wie Methoxy, Ethoxy, n-Propoxy, 1-Methylethoxy, n-Butoxy, 1-Methylpropoxy, 2-Methylpropoxy und 1,1-Dimethylethoxy,
insbesondere C₁-C₃-Alkoxy wie Methoxy, Ethoxy, i-Propoxy,
wobei diese Gruppen gegebenenfalls durch ein bis fünf Halogenatome wie Fluor, Chlor, Brom und Iod, vorzugsweise Fluor und Chlor oder C₁-C₄-Alkoxy wie vorstehend genannt substituiert sein können.

Die vorstehend definierte Gruppe -(Y)ₙ-S(O)ₘR⁷ steht beispielsweise für
C₁-C₄-Alkylthio wie Methylthio, Ethylthio, n-Propylthio, 1-Methylethylthio, n-Butylthio, 1-Methylpropylthio, 2-Methylpropylthio und 1,1-Dimethylethylthio, insbesondere Methylthio;
C₁-C₄-Alkylsulfinyl wie Methylsulfinyl, Ethylsulfinyl, n-Propylsulfinyl, 1-Methylethylsulfinyl, n-Butylsulfinyl, 1-Methylpropylsulfinyl, 2-Methylpropylsulfinyl und 1,1-Dimethylethylsulfinyl, insbesondere Methylsulfinyl;
C₁-C₄-Alkylsulfonyl wie Methylsulfonyl, Ethylsulfonyl, n-Propylsulfonyl, 1-Methylethylsulfonyl, n-Butylsulfonyl, 1-Methylpropylsulfonyl, 2-Methylpropylsulfonyl und 1,1-Dimethylethylsulfonyl, insbesondere Methylsulfonyl;
C₁-C₄-Alkoxysulfonyl wie Methoxysulfonyl, Ethoxysulfonyl, n-Propoxysulfonyl, 1-Methylethoxysulfonyl, n-Butoxysulfonyl, 1-Methylpropoxysulfonyl, 2-Methylpropoxysulfonyl und 1,1-Dimethylethoxysulfonyl, insbesondere Methoxysulfonyl;
N-C₁-C₄-Alkylsulfamoyl wie N-Methylsulfamoyl, N-Ethylsulfamoyl, N-n-Propylsulfamoyl, N-1-Methylethylsulfamoyl, N-n-Butylsulfamoyl, N-1-Methylpropylsulfamoyl, N-2-Methylpropylsulfamoyl und N-1,1-Dimethylethylsulfamoyl, insbesondere N-Methylsulfamoyl;
N-C₁-C₄-Alkylsulfinamoyl wie N-Methylsulfinamoyl, N-Ethylsulfinamoyl, N-n-Propylsulfinamoyl, N-1-Methylethylsulfinamoyl, N-n-Butylsulfinamoyl, N-1-Methylpropylsulfinamoyl, N-2-Methylpropylsulfinamoyl und N-1,1-Dimethylethylsulfinamoyl, insbesondere N-Methylsulfinamoyl;
Di-C₁-C₄-Alkylsulfamoyl wie Dimethylsulfamoyl, Diethylsulfamoyl, Dipropylsulfamoyl, Dibutylsulfamoyl, N-Methyl-N-ethylsulfamoyl, N-Methyl-N-propylsulfamoyl, N-Methyl-N-1-methylethylsulfamoyl, N-Methyl-N-1,1-Dimethylethylsulfamoyl, Di-1-Methylethylsulfamoyl, N-Ethyl-N-1-Methylethylsulfamoyl und N-Ethyl-N-1,1-dimethylethylsulfamoyl; insbesondere Dimethylsulfamoyl;
Di-C₁-C₄-Alkylsulfinamoyl wie Dimethylsulfinamoyl, Diethylsulfinamoyl, Dipropylsulfinamoyl, Dibutylsulfinamoyl, N-Methyl-N-ethylsulfinamoyl, N-Methyl-N-propylsulfinamoyl, N-Methyl-N-1-methylethylsulfinamoyl, N-Methyl-N-1,1-Dimethylethylsulfinamoyl, Di-1-Methylethylsulfinamoyl, N-Ethyl-N-1-Methylethylsulfinamoyl und N-Ethyl-N-1,1-dimethylethylsulfinamoyl; insbesondere Dimethylsulfinamoyl,
C₁-C₄-Alkylsulfinyloxy wie Methylsulfinyloxy, Ethylsulfinyloxy, n-Propylsulfinyloxy, 1-Methylethylsulfinyloxy, n-Butylsulfinyloxy, 1-Methylpropylsulfinyloxy, 2-Methylpropylsulfinyloxy und 1,1-Dimethylethylsulfinyloxy, insbesondere Methylsulfinyloxy;
C₁-C₄-Alkylsulfonyloxy wie Methylsulfonyloxy, Ethylsulfonyloxy, n-Propylsulfonyloxy, 1-Methylethylsulfonyloxy, n-Butylsulfonyloxy, 1-Methylpropylsulfonyloxy, 2-Methylpropylsulfonyloxy und 1,1-Dimethylethylsulfonyloxy, insbesondere Methylsulfonyloxy;
C₁-C₄-Alkylsulfinylamino wie Methylsulfinylamino, Ethylsulfinylamino, n-Propylsulfinylamino, 1-Methylethylsulfinylamino, n-Butylsulfinylamino, 1-Methylpropylsulfinylamino, 2-Methylpropylsulfinylamino und 1,1-Dimethylethylsulfinylamino, insbesondere Methylsulfinylamino;
C₁-C₄-Alkylsulfonylamino wie Methylsulfonylamino, Ethylsulfonylamino, n-Propylsulfonylamino, 1-Methylethylsulfonylamino, n-Butylsulfonylamino, 1-Methylpropylsulfonylamino, 2-Methylpropylsulfonylamino und 1,1-Dimethylethylsulfonylamino, insbesondere Methylsulfonylamino;
N-C₁-C₄-Alkylsulfinyl-N-methyl-amino wie N-Methylsulfinyl-N-methyl-amino, N-Ethylsulfinyl-N-methyl-amino, N-n-Propylsulfinyl-N-methyl-amino, N-1-Methylethylsulfinyl-N-methyl-amino, N-n-Butylsulfinyl-N-methyl-amino, N-1-Methylpropylsulfinyl-N-methyl-amino, N-2-Methylpropylsulfinyl-N-methyl-amino und N-1,1-Dimethylethylsulfinyl-N-methyl-amino, insbesondere N-Methylsulfinyl-N-methyl-amino;
N-C₁-C₄-Alkylsulfinyl-N-ethyl-amino wie N-Methylsulfinyl-N-ethylamino, N-Ethylsulfinyl-N-ethyl-amino, N-n-Propylsulfinyl-N-ethylamino, N-1-Methylethylsulfinyl-N-ethyl-amino, N-n-Butylsulfinyl-N-ethyl-amino, N-1-Methylpropylsulfinyl-N-ethyl-amino, N-2-Methylpropylsulfinyl-N-ethyl-amino und N-1,1-Dimethylethylsulfinyl-N-ethyl-amino, insbesondere N-Methylsulfinyl-N-ethyl-amino;
N-C₁-C₄-Alkylsulfonyl-N-methyl-amino wie N-Methylsulfonyl-N-methyl-amino, N-Ethylsulfonyl-N-methyl-amino, N-n-Propylsulfonyl-N-methyl-amino, N-1-Methylethylsulfonyl-N-methyl-amino, N-n-Butylsulfonyl-N-methyl-amino, N-1-Methylpropylsulfonyl-N-methyl-amino, N-2-Methylpropylsulfonyl-N-methyl-amino und N-1,1-Dimethylethylsulfonyl-N-methyl-amino, insbesondere N-Methylsulfonyl-N-methyl-amino;
N-C₁-C₄-Alkylsulfonyl-N-ethyl-amino wie N-Methylsulfonyl-N-ethylamino, N-Ethylsulfonyl-N-ethyl-amino, N-n-Propylsulfonyl-N-ethylamino, N-1-Methylethylsulfonyl-N-ethyl-amino, N-n-Butylsulfonyl-N-ethyl-amino, N-1-Methylpropylsulfonyl-N-ethyl-amino, N-2-Methylpropylsulfonyl-N-ethyl-amino und N-1,1-Dimethylethylsulfonyl-N-ethyl-amino, insbesondere N-Methylsulfonyl-N-ethyl-amino;
C₁-C₄-Halogenalkylthio wie Chlormethylthio, Dichlormethylthio, Trichlormethylthio, Fluormethylthio, Difluormethylthio, Trifluormethylthio, Chlorfluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2 fluorethylthio, 2,2,2-Trichlorethylthio und Pentafluorethylthio, insbesondere Trifluormethylthio.

Die vorstehend definierte Gruppe -(Y)ₙ-CO-R⁸ steht beispielsweise für
C₁-C₄-Alkylcarbonyl wie Methylcarbonyl, Ethylcarbonyl, n-Propylcarbonyl, 1-Methylethylcarbonyl, n-Butylcarbonyl, 1-Methylpropylcarbonyl, 2-Methylpropylcarbonyl und 1,1-Dimethylethylcarbonyl, insbesondere Methylcarbonyl;
C₁-C₄-Alkoxycarbonyl wie Methoxycarbonyl, Ethoxycarbonyl, n-Propoxycarbonyl, 1-Methylethoxycarbonyl, n-Butoxycarbonyl, 1-Methylpropoxycarbonyl, 2-Methylpropoxycarbonyl und 1,1-Dimethylethoxycarbonyl, insbesondere Methoxycarbonyl;
N-C₁-C₄-Alkylcarbamoyl wie N-Methylcarbamoyl, N-Ethylcarbamoyl, N-n-Propylcarbamoyl, N-1-Methylethylcarbamoyl, N-n-Butylcarbamoyl, N-1-Methylpropylcarbamoyl, N-2-Methylpropylcarbamoyl und N-1,1-Dimethylethylcarbamoyl, insbesondere N-Methylcarbamoyl;
Di-C₁-C₄-Alkylcarbamoyl wie Dimethylcarbamoyl, Diethylcarbamoyl, Dipropylcarbamoyl, Dibutylcarbamoyl, N-Methyl-N-ethylcarbamoyl, N-Methyl-N-propylcarbamoyl, N-Methyl-N-1-methylethylcarbamoyl, N-Methyl-N-1,1-Dimethylethylcarbamoyl, Di-1-Methylethylcarbamoyl, N-Ethyl-N-1-Methylethylcarbamoyl und N-Ethyl-N-1,1-dimethyl ethylcarbamoyl; insbesondere Dimethylcarbamoyl;
C₁-C₄-Alkylcarbonyloxy wie Methylcarbonyloxy, Ethylcarbonyloxy, n-Propylcarbonyloxy, 1-Methylethylcarbonyloxy, n-Butylcarbonyloxy, 1-Methylpropylcarbonyloxy, 2-Methylpropylcarbonyloxy und 1,1-Dimethylethylcarbonyloxy, insbesondere Methylcarbonyloxy; C₁-C₄-Alkylcarbonylamino wie Methylcarbonylamino, Ethylcarbonylamino, n-Propylcarbonylamino, 1-Methylethylcarbonylamino, n-Butylcarbonylamino, 1-Methylpropylcarbonylamino, 2-Methylpropylcarbonylamino und 1,1-Dimethylethylcarbonylamino, insbesondere Methylcarbonylamino;
N-C₁-C₄-Alkylcarbonyl-N-methyl-amino wie N-Methylcarbonyl-N-methyl-amino, N-Ethylcarbonyl-N-methyl-amino, N-n-Propylcarbonyl-N-methyl-amino, N-1-Methylethylcarbonyl-N-methyl-amino, N-n-Butylcarbonyl-N-methyl-amino, N-1-Methylpropylcarbonyl-N-methyl-amino, N-2-Methylpropylcarbonyl-N-methyl-amino und N-1,1-Dimethylethylcarbonyl-N-methyl-amino, insbesondere N-Methylcarbonyl-N-methyl-amino.

Z steht beispielsweise für:
5- oder 6-gliedriger heterocyclischer, gesättigter oder ungesättigter Rest, enthaltend ein bis drei Heteroatome, ausgewählt aus der Gruppe Sauerstoff, Schwefel oder Stickstoff, beispielsweise fünfring Heteroaromaten wie 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, 1,2,4-Thiadiazol-3-yl, 1,2,4-Thiadiazol-5-yl, 1,3,4-Thiadiazol-2-yl, 1,2,3-Thiadiazol-4-yl, 1,2,3-Thiadiazol-5-yl, 1,2,5-Thiadiazol-3-yl, 1,2,4-Triazol-3-yl, , 1,3,4-Triazol-2-yl,1,2,3-Triazol-4-yl, 1,2,3-Triazol-5-yl, 1,2,4-Triazol-5-yl, Tetrazol-5-yl, insbesondere 2-Thiazolyl und 3-Isoxazolyl;
sechsring Heteroaromaten wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-5-yl und 1,2,4-Triazin-3-yl, 1,2,4-Triazin-6-yl, 1,2,4,5-Tetrazin-3-yl;
5- bis 6-gliedrige, gesättigte oder teilweise ungesättigte Heterocyclen, enthaltend ein bis drei Stickstoffatome und/oder ein oder zwei Sauerstoff- oder Schwefelatom wie 2-Tetrahydrofuranyl, 3-Tetrahydrofuranyl, 2-Tetrahydrothienyl, 3-Tetrahydrothienyl, Tetrahydrothiopyran-2-yl, Tetrahydrothiopyran-3-yl, Tetrahydrothiopyran-4-yl, 1,3-Dithiolan-2-yl, 1,3-Dithiolan-4-yl, 1-3-Dithian-2-yl, 1,3-Dithian-4-yl, 5,6-Dihydro-4H-1,3-thiazin-2-yl, 1,3-Oxathiolan-2-yl, 1,3-Oxathian-2-yl, 1-Pyrrolidinyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl,1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl,2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl,2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl. 2,3-Dihydrooxazol-3-yl, 2,3-Dihydrooxazol-4-yl, 2,3-Dihydrooxazol-5-yl, 4,5-Dihydrooxazol-2-yl, 4,5-Dihydrooxazol-4-yl, 4,5-Dihydrooxazol-5-yl, 1,3-Dioxolan-2-yl, 1,3-Dioxolan-4-yl, 1,3-Dioxan-5-yl, 1,4-Dioxan-2-yl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydro-triazin-2-yl und 1,2,4-Tetrahydrotriazin-3-yl, insbesondere 2-Tetrahydrofuranyl, 1,3-Dioxolan-2-yl und 1,3-Dioxan-2-yl,
der gegebenenfalls durch
Halogen wie vorstehend genannt, insbesondere Fluor oder Chlor,
Cyano, Nitro,
eine Gruppe -COR⁸, beispielsweise Alkylcarbonyl wie vorstehend genannt, Alkoxycarbonyl wie vorstehend genannt, N-Alkylcarbamoyl wie vorstehend genannt, Dialkylcarbamoyl wie vorstehend genannt; C₁-C₄-Alkyl wie vorstehend genannt,
C₁-C₄-Halogenalkyl wie beispielsweise Chlormethyl, Difluormethyl, Dichlormethyl, Trifluormethyl, Trichlormethyl, Chlordifluormethyl, 1-Fluorethyl, 2-Fluorethyl, 2,2-Difluorethyl, 1,1,2,2-Tetrafluorethyl, 2,2,2-Trifluorethyl, 2-Chlor-1,1,2-trifluorethyl und Pentafluorethyl, Decafluorbutyl, 1,1-Bis-trifluormethyl-2,2,2-trifluorethyl, bevorzugt Difluormethyl, Trifluormethyl, Trichlormethyl und Chlordifluormethyl;
C₃-C₈-Cycloalkyl, wie beispielsweise Cyclopropyl, Cyclobutyl, Cyclopentyl, Cyclohexyl, Cycloheptyl, Cyclooctyl, insbesondere Cyclopropyl und Cyclohexyl;
C₁-C₄-Alkoxy wie vorstehend genannt,
C₁-C₄-Halogenalkoxy wie beispielsweise Chlormethoxy, Dichlormethoxy, Trichlormethoxy, Fluormethoxy, Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere C₁-C₃-Halogenalkoxy wie 2,2,2-Trifluorethyloxy und 2-Chlor-2,2-difluorethoxy;
C₁-C₄-Alkylthio wie vorstehend genannt,
C₁-C₄-Halogenalkylthio wie vorstehend genannt,
Di-C₁-C₄-Alkylamino wie beispielsweise Dimethylamino, Diethylamino, Dipropylamino, Dibutylamino, N-Methyl-N-ethylamino, N-Methyl-N-propylamino, N-Methyl-N-1-methylethylamino, N-Methyl-N-1,1-Dimethylethylamino, Di-1-Methylethylamino, N-Ethyl-N-1-methylethylamino und N-Ethyl-N-1,1-dimethylethylamino;
gegebenenfalls substituiertes Phenyl
oder eine Oxogruppe, die gegebenenfalls auch in der tautomeren Form als Hydroxygruppe vorliegen kann, substituiert ist, beispielsweise Thiazolin-4,5-dion-2-yl, 3-Oxo-3H-1,2,4-dithiazolyl oder 2-Oxo-2H-1,3,4-dithiazolyl.

Benzokondensierte 5- oder 6-Ring-Heteroaromaten sind beispielsweise Benzofuranyl, Benzothienyl, Indolyl, Benzoxazolyl, Benzisoxazolyl, Benzthiazolyl, Benzisothiazolyl, Benzpyrazolyl, Indazolyl, 1,2,3-Benzothiadiazolyl, 2,1,3-Benzothiadiazolyl, Benzotriazolyl, Benzofuroxanyl, Chinolinyl, Isochinolinyl, Cinnolinyl, Chinazolinyl, Chinoxalinyl oder Phthalazinyl. Bei-spiele für besonders bevorzugte Verbindungen der allgemeinen Formel I sind in der folgenden Tabelle 1 zusammengestellt.

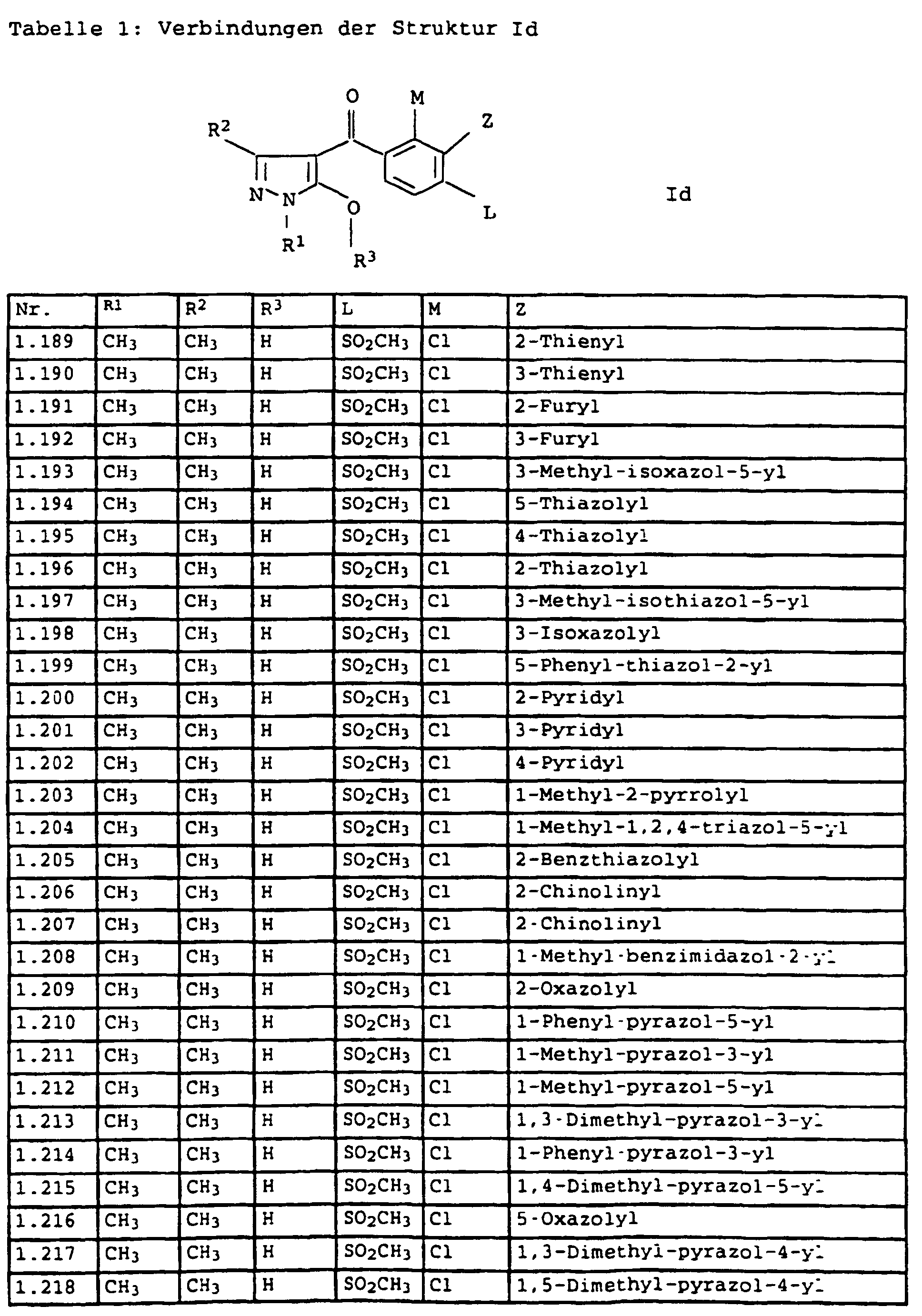

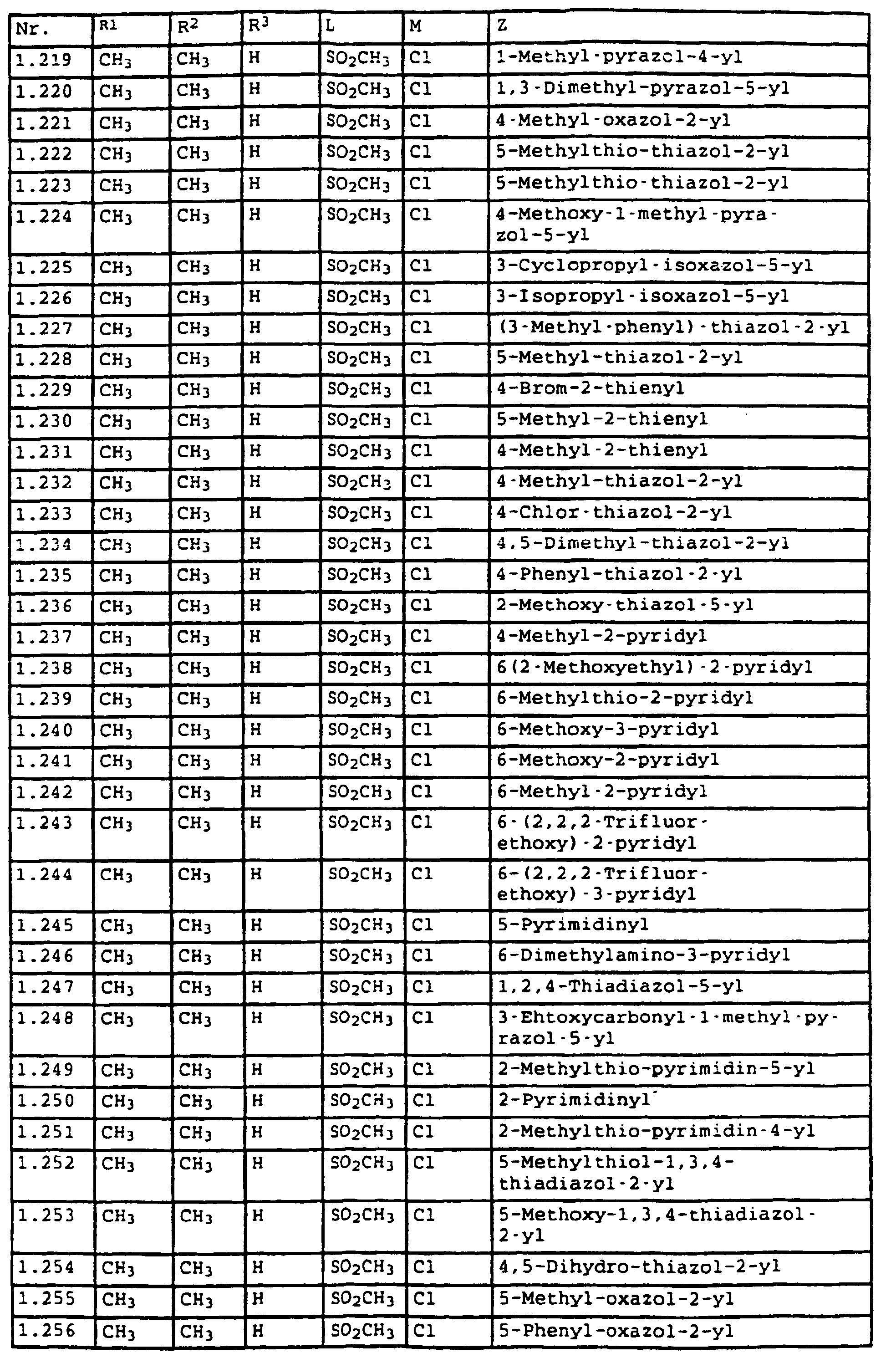

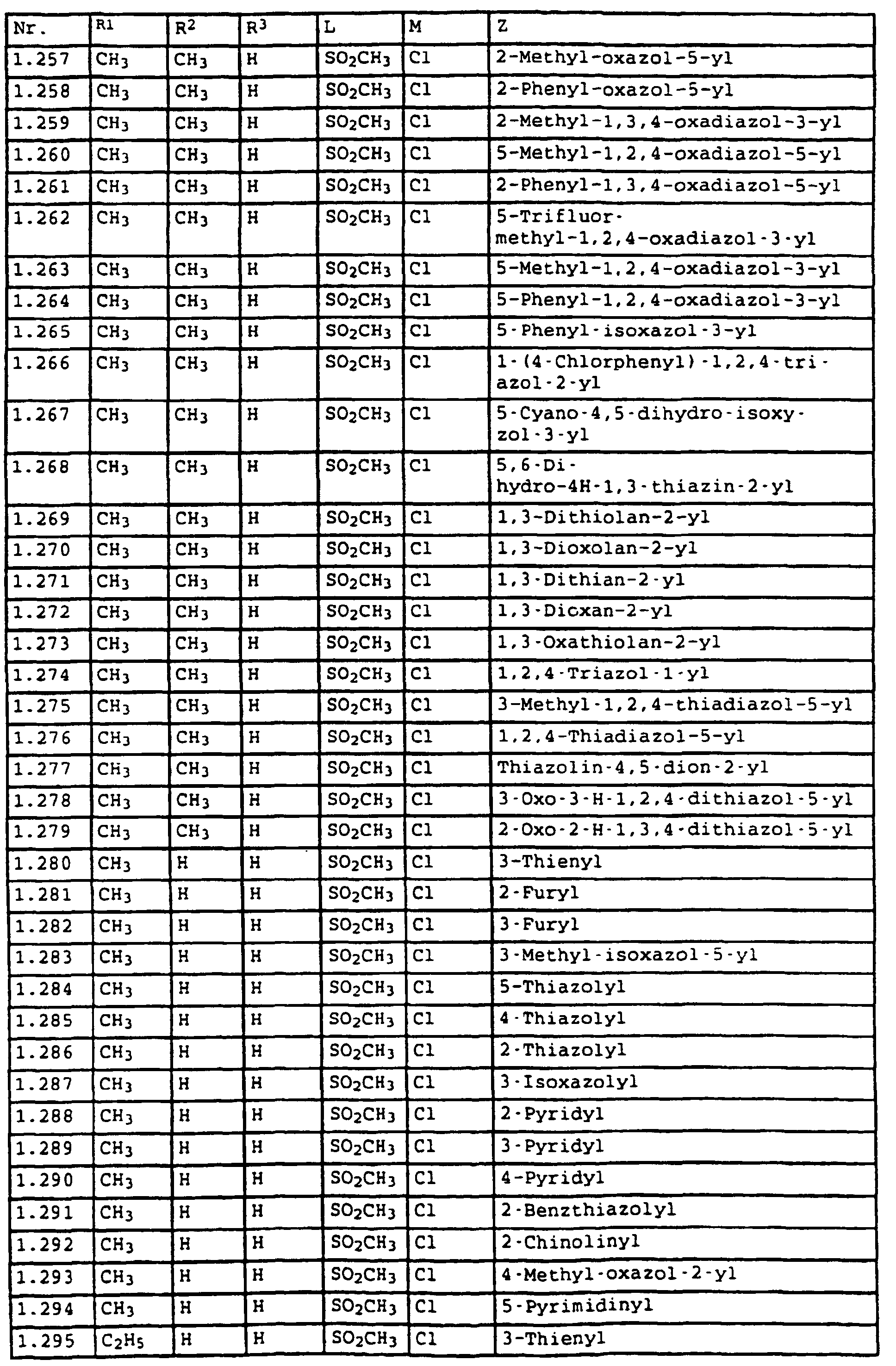

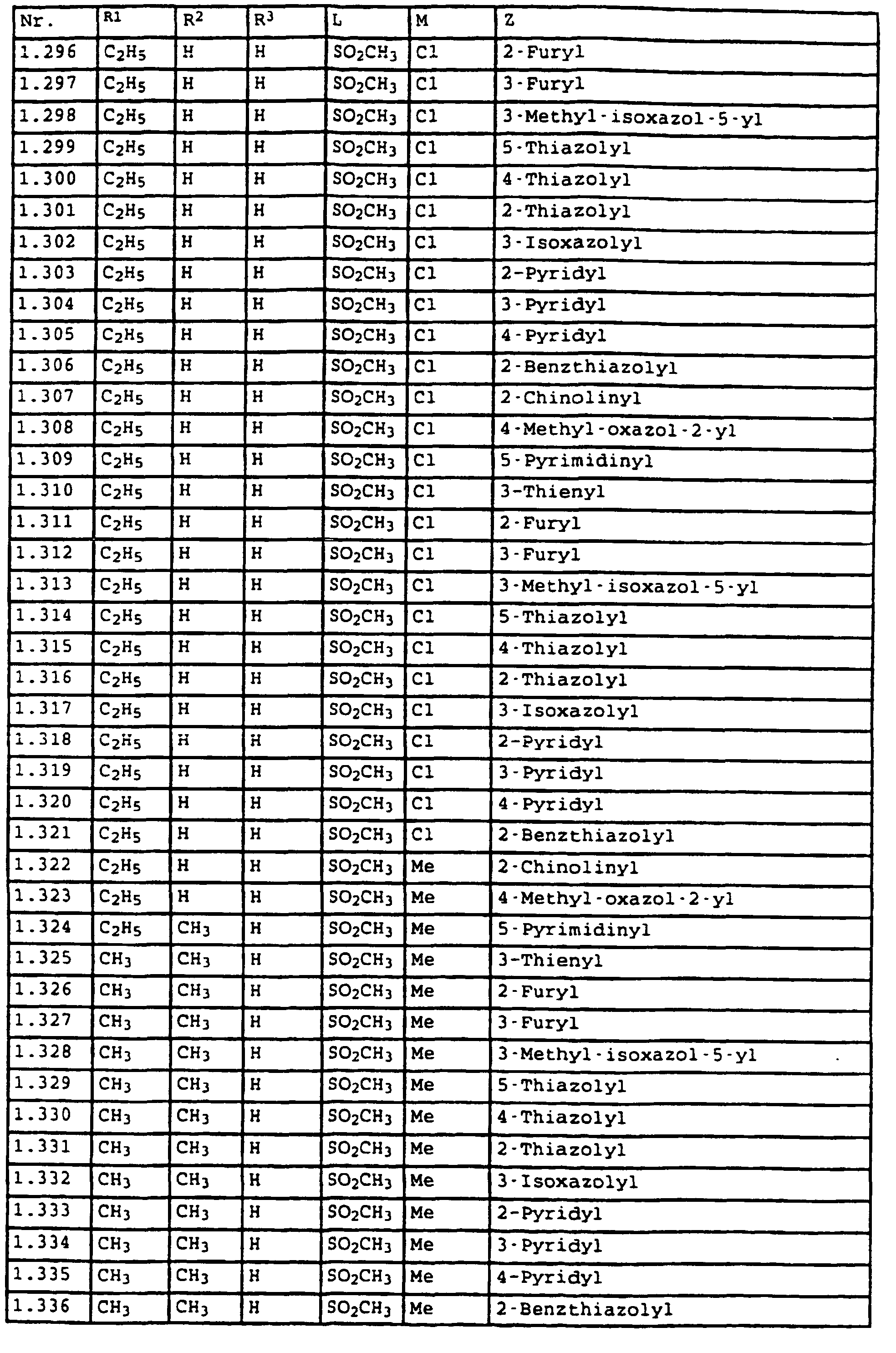

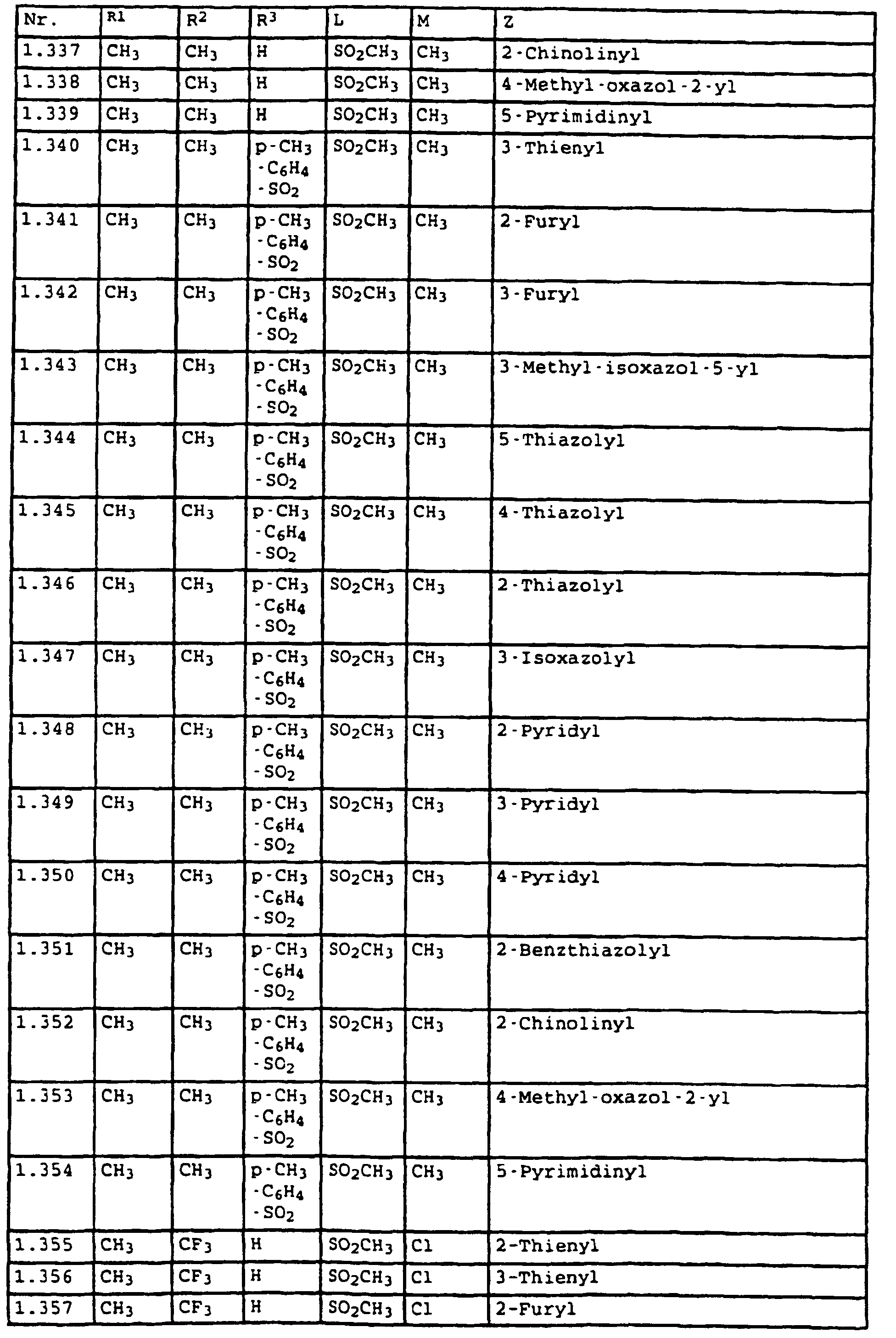

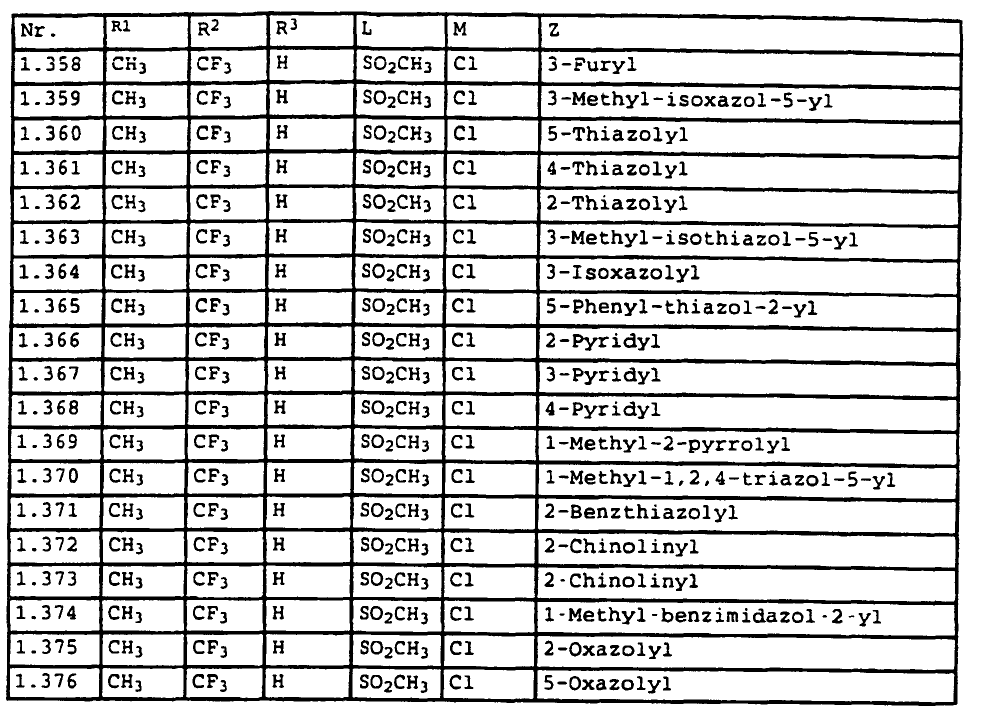

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

Unter Berücksichtigung der Vielseitigkeit der Applikationsmethoden können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N. rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen I auch in Kulturen, die durch Züchtung einschließlich gentechnischer Methoden gegen die Wirkung von Herbiziden tolerant sind, verwandt werden.

Die Applikation der herbiziden Mittel bzw. der Wirkstoffe kann im Vorauf lauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, vernebeln, Verstäuben, Verstreuen oder Gießen angewendet werden. Die Anwendungsformen richten sich nach den verwendungszwecken; sie sollten in jedem Fall möglichst die feinste Verteilung der erfindungsgemäßen Wirkstoffe gewährleisten.

Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon oder stark polare Lösungsmittel, wie N-Methylpyrrolidon oder Wasser in Betracht.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z. B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, Wirkstoff. Die Wirkstoffe werden dabei in einer Reinheit von 90 % bis 100 %, vorzugsweise 95 % bis 100 % (nach NMR-Sektrum) eingesetzt.

Die erfindungsgemäßen Verbindungen I können beispielsweise wie folgt formuliert werden:
- I: 20 Gewichtsteile der Verbindung Nr. 1.28 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew. % des Wirkstoffs enthält.
- II: 20 Gewichtsteile der Verbindung Nr. 1.28 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenyl und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
- III: 20 Gewichtsteile des Wirkstoffs Nr. 1.28 werden in einer Mischung gelöst, die aus 25 Gewichtsteilen Cyclohexanon, 65 Gewichtsteilen einer Mineralölfraktion vom Siedepunkt 1.280 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
- IV: 20 Gewichtsteile des Wirkstoffs Nr. 1.28 werden mit 3 Gewichtsteilen des Natriumsalzes der Diisobutylnaphthalin-a-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines verteilen der Mischung in 20 000 Gewichtsteilen Wasser enthält man eine Spritzbrühe, die 0,1 Gew. % des Wirkstoffs enthält.
- V: 3 Gewichtsteile des Wirkstoffs Nr. 1.28 werden mit 97 Gewichtsteilen feinteiligem Kaolin vermischt. Man erhält auf diese Weise ein Stäubemittel, das 3 Gew. % des Wirkstoffs enthält.
- VI: 20 Gewichtsteile des Wirkstoffs Nr. 1.28 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
- VII: 1 Gewichtsteil der Verbindung Nr. 1.28 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
- VIII: 1 Gewichtsteil der Verbindung Nr. 1.28 wird in einer Mischung gelöst, die aus 80 Gewichtsteilen Cyclohexanon und 20 Gewichtsteilen Emulphor EL besteht. Man erhält ein stabiles Emulsionskonzentrat.

Zur Verbreiterung des Wirkungsspektrums und zur Erzielung synergistischer Effekte können die Pyrazolyl-benzoylderivate I mit zahlreichen Vertretern anderer herbizider oder wachstums-regulierender Wirkstoffgruppen gemischt und gemeinsam ausgebracht werden. Beispielsweise kommen als Mischungspartner Diazine, 4H-3, 1-Benzoxazinderivate, Benzothiadiazinone, 2, 6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z. B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a.S.).

### Anwendungsbeispiele

Die herbizide Wirkung der Pyrazolyl-benzoylderivate der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt- bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung werden die Testpflanzen je nach wuchsform erst bis zu einer Wuchshöhe von 3 bis 15 cm angezogen und erst dann mit den in Wasser suspendierten oder emulgierten wirkstoffen behandelt. Die Testpflanzen werden dafür entweder direkt gesät und in den gleichen Gefäßen aufgezogen oder sie werden erst als Keimpflanzen getrennt angezogen und einige Tage vor der Behandlung in die Versuchsgefäße verpflanzt.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

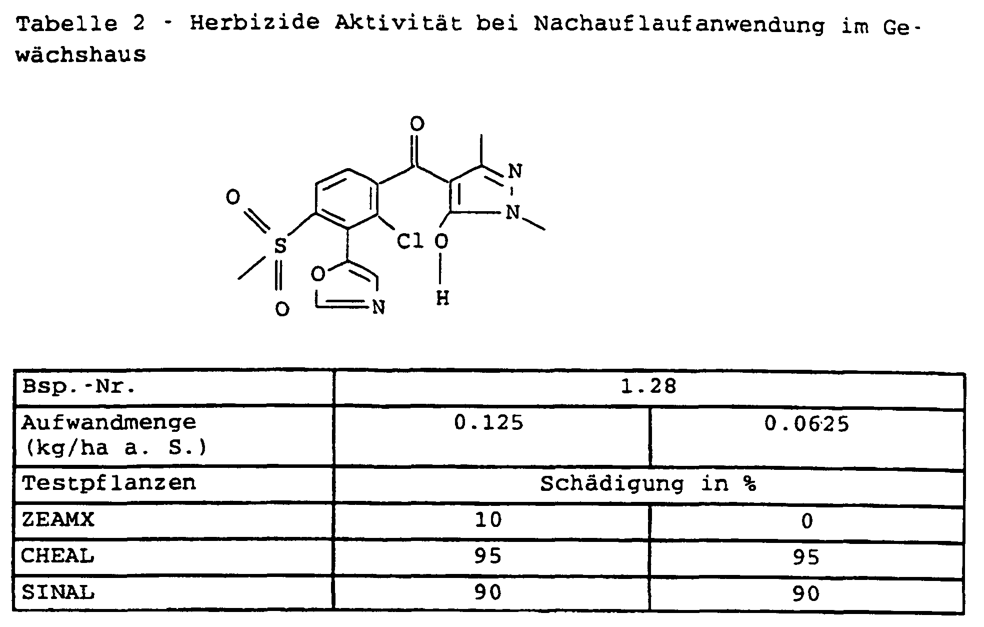

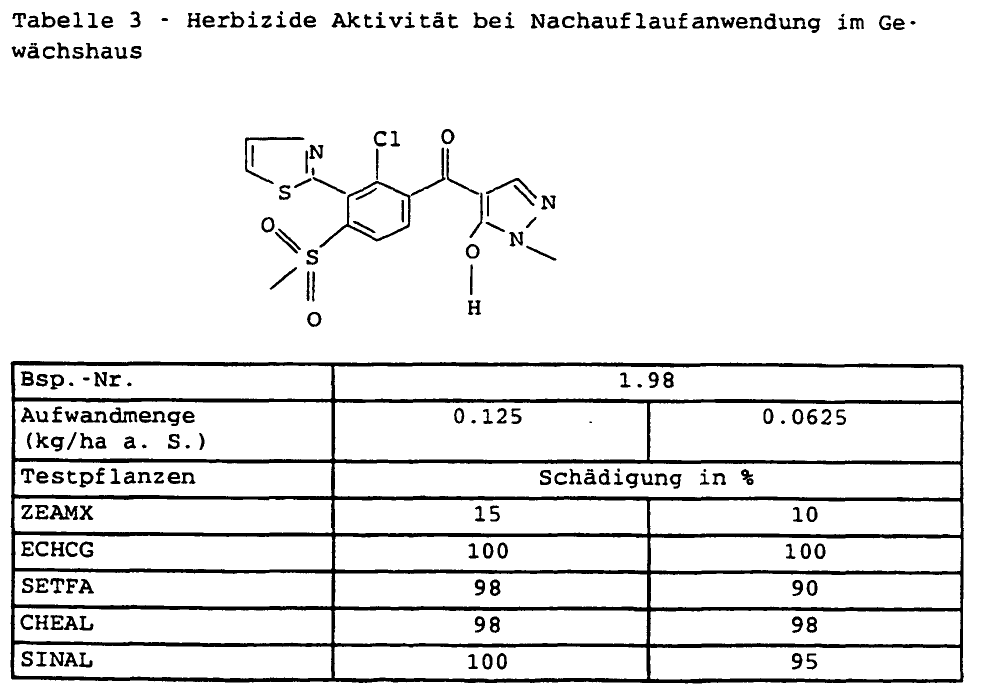

### Herstellungsbeispiele

### A) Herstellung der Ausgangsstoffe

### 1. 2-Chlor-3-formyl-4-methylsulfonyl-benzoesäuremethylester

a. Zu einer Suspension von 286 g (2.14 mol) Aluminiumtri- chlorid in 420 ml 1,2-Dichlorethan wurde bei 15-20°C eine Lösung von 157 g (2 mol) Acetylchlorid in 420 mol 1,2-Dichlorethan getropft. Anschließend wurde eine Lösung von 346 g (2 mol) 2-Chlor-6-methylthio-toluol in 1 l 1,2-Dichlorethan zugetropft. Nach 12 Stunden Nachrühren wurde das Reaktionsgemisch in eine Mischung aus 3 l Eis und 1 l konz. HCl gegossen. Es wurde mit Methylenchlorid extrahiert, die organische Phase mit Wasser gewaschen, mit Natriumsulfat getrocknet und eingeengt. Der Rückstand wurde im Vakuum destilliert.
   Man erhielt 256 g (60 % d.Th.) 2-Chlor-3-methyl-4-me- thylthio-acetophenon,
   Fp.: 46°C
b. 163 g (0.76 mol) 2-Chlor-3-methyl-4-methylthio-acezozhe- non wurden in 1,51 Eisessig gelöst , mit 18,6 g Natrium- wolframat versetzt und unter Kühlung 173,3 g 30 %ige Wasserstoffperoxidlösung zugetropft. Es wurde 2 Tage nachgerührt und anschließend mit Wasser verdünnt. Der ausgefallene Feststoff wurde abgesaugt, mit Wasser ge- waschen und getrocknet.
   Man erhielt 164 g (88% d. Th.) 2-Chlor-3-methyl-4-methyl- sulfonyl-acetophenon, Fp.: 110-111°C
c. 82 g (0.33 mol) 2-Chlor-3-methyl-4-methylsulfonyl-aceto- phenon wurden in 700 ml Dioxan gelöst und bei Raumtempe- ratur mit 1 1 einer 12,5 %igen Natriumhypochloritlosung versetzt. Anschließend wurde 1 Stunde bei 80°C nach- gerührt. Nach dem Abkühlen bildeten sich zwei Phasen, von denen die untere mit Wasser verdünnt und schwach ange- säuert wurde. Der ausgefallene Feststoff wurde mit Wasser nachgewaschen und getrocknet.
   Man erhielt 60 g (73 % d.Th) 2-Chlor-3-methyl-4-methyl- sulfonyl-benzoesäure, Fp.: 230-231°C.
d. 100 g (0.4 mol) 2-Chlor-3-methyl-4-methylsulfonyl-benzoe- säure wurden in 1 l Methanol gelöst und bei Rückfluß- temperatur 5 Stunden mit HCl begast. Anschließend wird eingeengt.
   Man erhielt 88.5 g (84 % d.Th.) 2-Chlor-3-methyl-4-me- thylsulfonyl-benzoesäuremethylester, Fp.: 107-108°C.
e. 82 g (0.31 mol) 2-Chlor-3-methyl-4-methylsulfonyl-benzoe- säuremethylester werden in 21 Tetrachlormethan gelöst und unter Belichtung portionsweise mit 56 g (0.31 mol) N-Bromsuccinimid versetzt. Das Reaktionsgemisch wurde filtriert, das Filtrat eingeengt und der Rückstand in 200 ml Methyl-tert.-butylether aufgenommem. Die Lösung wird mit Petrolether versetzt, der ausgefallene Feststoff abgesaugt und getrocknet.
   Man erhielt 74,5 g (70 % d.Th) 3-Brommethyl-2-chlor-4- methylsulfonyl-benzoesäuremethylester, Fp.: 74-75°C.
f. Eine Lösung von 41 g (0.12 mol) 3-Brommethyl-2-chlor- 4-methylsulfonyl-benzoesäuremethylester in 250 ml Aceto- nitril wurde mit 42,1 g (0.36 mol) N-Methylmorpholin-N- oxid versetzt. Der Ansatz wurde 12 Stunden bei Raumtempe- ratur nachgerührt, anschließend eingeengt und der Rück- stand in Essigester aufgenommen. Die Lösung wurde mit Wasser extrahiert, mit Natriumsulfat getrocknet und ein- geengt.
   Man erhielt 31,2 g (94 % d.Th.) 2-Chlor-3-formyl-4- methylsulfonyl-benzoesäuremethylester, Fp.: 98-105°C

### 2. 2-Chlor-4-methylsulfonyl-3-(trifluormethylsulfonyl)oxy-benzoesäure-methylester

a. 101 g (0.41 mol) 2-Chlor-3-hydroxy-4-methylsulfonyl-ben- zoesäure werden in 1,31 Methanol gelöst und unter Rück- fluß 4 Stunden mit HCl begast. Die Lösung wurde einge- engt, der Rückstand mit Dichlormethan aufgenommen und mit K₂CO₃-Lösung extrahiert. Die wäßrige Phase wurde mit ver- dünnter Salzsäure auf pH 7 eingestellt und mit Dichlor- methan gewaschen. Anschließend wurde auf pH 1 angesäuert und das Produkt mit Dichlormethan extrahiert.
   Man erhielt 76,2 g (71 % d.Th.) 2-Chlor-3-hydroxy-4- methylsulfonyl-benzoesäuremethylester.
b. Eine Lösung aus 76 g (0,29 mol) 2-Chlor-3-hydroxy-4-me- thylsulfonyl-benzoesäuremethylester und 68 g Pyridin in 700 ml Dichlormethan wurde bei -20°C mit 89 g (0.32 mol) Trifluormethansulfonsäureanhydrid versetzt. Die Lösung wurde 12 Stunden bei Raumtemperatur nachgerührt, mit Dichlormethan verdünnt und mit Wasser extrahiert. Die or- ganische Phase wurde über Magnesiumsulphat getrocknet und eingeengt.
   Man erhielt 94 g (82 % d.Th) 2-Chlor-4-methyl- sulfonyl-3-(trifluormethylsulfonyl)oxy-benzoesäure-me- thylester, Fp.: 69°C.

### B) Herstellung der Zwischenprodukte

### 1. 3-(3-Isopropylisoxazol-5-yl)-4-methylsulfonyl-benzoesäuremethylester

a. 30 g (102 mmol) 3-Brom-4-methylsulfonyl-benzoesäureme- thylester, 90 mg Palladiumdichlorid und 240 mg Triphenyl- phosphin in 200 ml Diethylamin und 60 ml Dimethylformamid werden mit 10 g (102 mmol) (Trimethylsilyl)-acetylen und 180 mg Kupfer-I-jodid versetzt und 4,5 Stunden bei 40°C gerührt. Anschließend wurde noch 12 Stunden bei Raumtem- peratur nachgerührt. Das Reaktionsgemisch wurde fil- triert, das Filtrat eingeengt und der Rückstand über Kieselgel mit Toluol als Laufmittel chromatographiert.
   Man erhielt 17,3g (55% d.Th.) 4-Methylsulfonyl-3-(tri- methylsilyl)ethinyl-benzoesäuremethylester als Öl.
b. 25 g 4-Methylsulfonyl-3-(trimethylsilyl)ethinyl-benzoe- säuremethylester werden mit 100 ml Methanol und 0,9 g Ka- liumkarbonat 18 Stunden bei Raumtemperatur gerührt. Anschließend wurde vom Feststoff abgesaugt, eingeengt und mit Essigester/Wasser extrahiert. Die organische Phase wurde über Natriumsulfat getrocknet und eingeengt.
   Man erhielt 15 g (79 % d.Th.) 4-Methylsulfonyl-3-ethinyl- benzoesäure-methylester, Fp.: 95-98°C.
c. 13,5 g (57 mmol) 4-Methylsulfonyl-3-ethinyl-benzoesäure- methylester werden in 50 ml Dichlormethan gelöst, mit 5,2g (60 mmol) Isobutyraldehydoxim versetzt und 41 g einer 12,5 %igen Natriumhypochloritlösung zugetropft. Anschließend wurde 24 Stunden bei Raumtemperatur nach- gerührt. Der Reaktionsansatz wurde anschließend mit Dichlormethan/Wasser extrahiert, die organische Phase eingeengt und der Rückstand über Kieselgel mit Toluol/- Essigester als Laufmittel chromatographiert.
Man erhielt 8,8 g (48 % d.Th) 3-(3-Isopropyl- isoxazol-5-yl)-4-methylsulfonyl-benzoesäuremethylester, Fp.: 102-104°C.

### 2. 2-Chlor-3-(isoxazol-3-yl)-4-methylsulfonyl-benzoesäuremethylester

a. 15 g ( 54 mmol) 2-Chlor-3-formyl-4-methylsulfonyl-benzoe- säuremethylester (Beisp. A.1.) und 4,2 g ( 60 mmol) Hydroxylaminhydrochlorid werden mit 300 ml Methanol ge- rührt und eine Lösung von 3,18 g (30 mmol) Natrium- carbonat in 80 ml Wasser zugetropft. Die Reaktionsmi- schung wird über Nacht bei Raumtemperatur gerührt, an- schließend wird das Methanol abdestilliert und der Ansatz mit Ether/Wasser extrahiert. Die Etherphase wird mit Na- triumsulfat getrocknet und eingeengt.
   Man erhält 14,4 g (91% d.Th.) 2-Chlor-3-hydroxyimino- methyl-4-methylsulfonyl-benzoesäuremethylester, Fp.: 126-128 °C.
b. 5,3 g ( 18 mmol) 2-Chlor-3-hydroxyiminomethyl-4-methyl- sulfonyl-benzoesäuremethylester werden in 50 ml Dichlor- methan gelöst und bei 0-5°C 30 Minuten lang Acetylen ein- geleitet. Anschließend wird mit mit einer Spatelspitze Natriumacetat versetzt und 15ml einer 10%igen Natrium- hypochlorit-Lösung bei 10°C unter weiterer Acetylen-Ein- leitung zutropft. Nach beendeter Zugabe wird für weitere 15 Minuten Acetylen bei 10°C eingeleitet und anschließend 12 Stunden nachgerührt. Danach werden die Phasen ge- trennt, die organische Phase mit Wasser gewaschen, mit Natriumsulfat getrocknet und eingeengt.
   Man erhält 4,8 g (84 % d.Th.) 2-Chlor-3-(isoxazol-3-yl)-4-methylsulfonyl-benzoesäure- methylester, Fp.: 145-147°C.

### 3. 2-Chlor-3-(thiazol-2-yl)-4-methylsulfonyl-benzoesäuremethylester

33 g (88 mmol) 2-(Tributylstannyl)-thiazol, 17,5 g (44 mmol) 2-Chlor-4-methylsulfonyl-3-(trifluormethylsulfonyl)oxy-benzoesäure-methylester (Beisp. A.2.), 5,8 g Lithiumchlorid, 1g Tetrakis-(triphenylphosphin)-palladium-(0), eine Spatelspitze 2,6-Di-tert.-butyl-4-methyl-phenol und 200 ml 1,4-Dioxan werden in einem Autoklaven 3 Stunden bei 140 °C unter Eigendruck gerührt. Nach dem Abkühlen wird die Reaktionsmischung über eine Kieselgelschicht abfiltriert, mit Methyl-tert.-butylether nachgewaschen und eingeengt. Der Rückstand wird über Kieselgel mit Toluol/Essigester als Laufmittel chromatographiert.

Man erhält 9,1 g (62,6% d.Th.) 2-Chlor-3-(thiazol-2-yl)-4-methylsulfonyl-benzoesäuremethylester, Fp.: 135-138 °C.

### 4. 2-Chlor-3-(oxazol-5-yl)-4-methylsulfonyl-benzoesäuremethylester

25 g (0,09 mol) 2-Chlor-3-formyl-4-methylsuflonyl-benzoesäuremethylester (Beisp. A.1.), 17,6 g (0,09 mol) Tosylmethylenisocyanid und 6,2 g (0,045 mol) fein gepulvertes Kaliumcarbonat werden mit 450 ml Methanol 5 Stunden bei RückfluBtemperatur gerührt. Anschließend wird das Lösungsmittel abgezogen, der Rückstand in Essigester aufgenommen und mit Wasser extrahiert. Die Essigesterphase wird mit Natriumsulfat getrocknet und eingeengt.

Man erhält 24,7 g (87 % d.Th.) 2-Chlor-3-(oxazol-5-yl)-4-methylsulfonylbenzoesäuremethylester, ¹H-NMR (CDCl₃)
δ: 8,24 (d, 1H), 8,15 (s, 1H), 8,01 (d, 1H), 7,40 (s, 1H), 4,0 (s,3H), 2,96 (s,3H)

In analoger Weise werden die in der nachfolgenden Tabelle aufgeführten Zwischenprodukte erhalten:

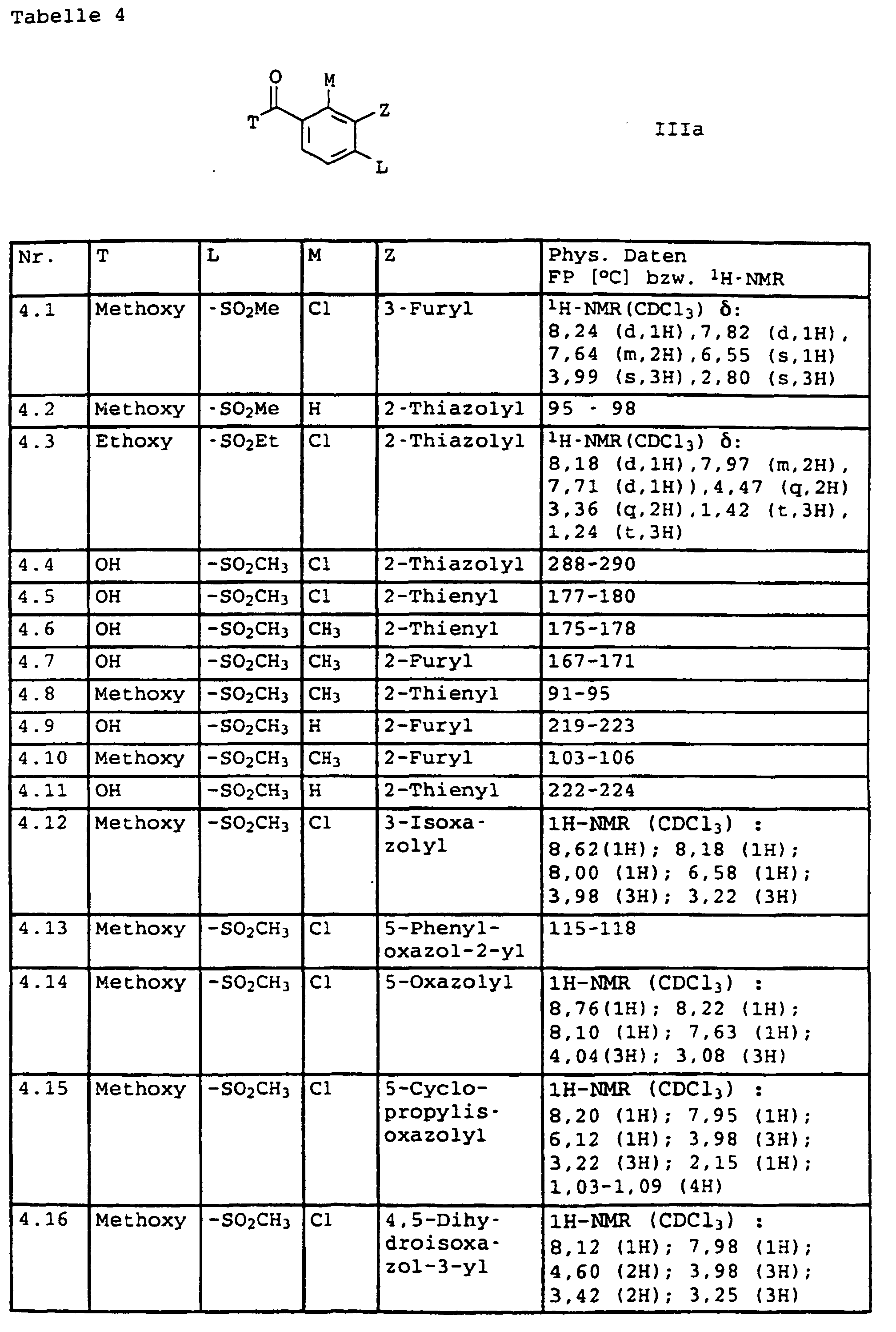

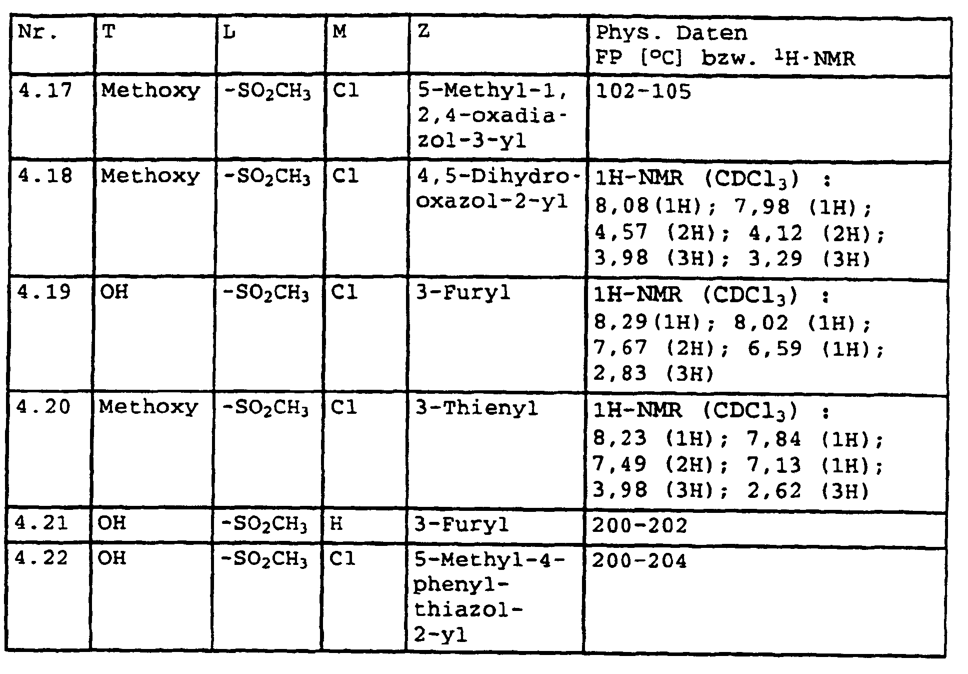

### Tabelle 5

### C) Herstellung der Endprodukte

### 1. 1,3-Dimethyl-4-[2-Chlor-4-methylsulfonyl-3-(oxazol-5-yl)-benzoyl]-5-hydroxy-pyrazol (Beispiel 1.28)

a. 1,22 g (10,9 mmol) 1,3-Dimethyl-5-hydroxypyrazol und 1,1 g (10,9 mmol) Triethylamin werden in 75 ml Aceto- nitril gelöst und bei 0°C mit 3,5 g (10,9 mmol) 2-Chlor-4-methylsulfonyl-3-(oxazol-5-yl)-benzoyl- chlorid in 50 ml Acetonitril versetzt. Es wird 1 Stunde bei 0°C nachgerührt und anschließend bei Raumtemperatur 4,45 g (44 mmol) Triethylamin und 0,61 g (7,2 mmol) Acetocyanhydrin zugetropft. Die Lösung wird 12 Stunden bei Raumtemperatur gerührt. Zur Aufarbeitung wird zunächst mit verdünnter Salz- säure versetzt und mit Methyl-tert.-butylether extra- hiert. Die Etherphase wird dann mit 5 % Kaliumcarbo- natlösung extrahiert. Aus der wäßrigen Phase wird nach dem Ansäuern mit Salzsäure das Produkt mit Es- sigester extrahiert. Die Essigesterphase wird mit Na- triumsulfat getrocknet und eingeengt.
   Man erhält 1,2 g Rohprodukt, das Säulenchromato- graphisch gereinigt wird.
   Man erhält 0,4 g (27 % d.Th.)
   1,3-Dimethyl-4-(2-Chlor-4-methyl- sulfonyl-3-(oxazol-5-yl)-benzoyl]-5-hydroxy-pyrazol, Fp.: 236 - 241°C.

In analoger Weise werden die in der nachfolgenden Tabelle aufgeführten Verbindungen erhalten:

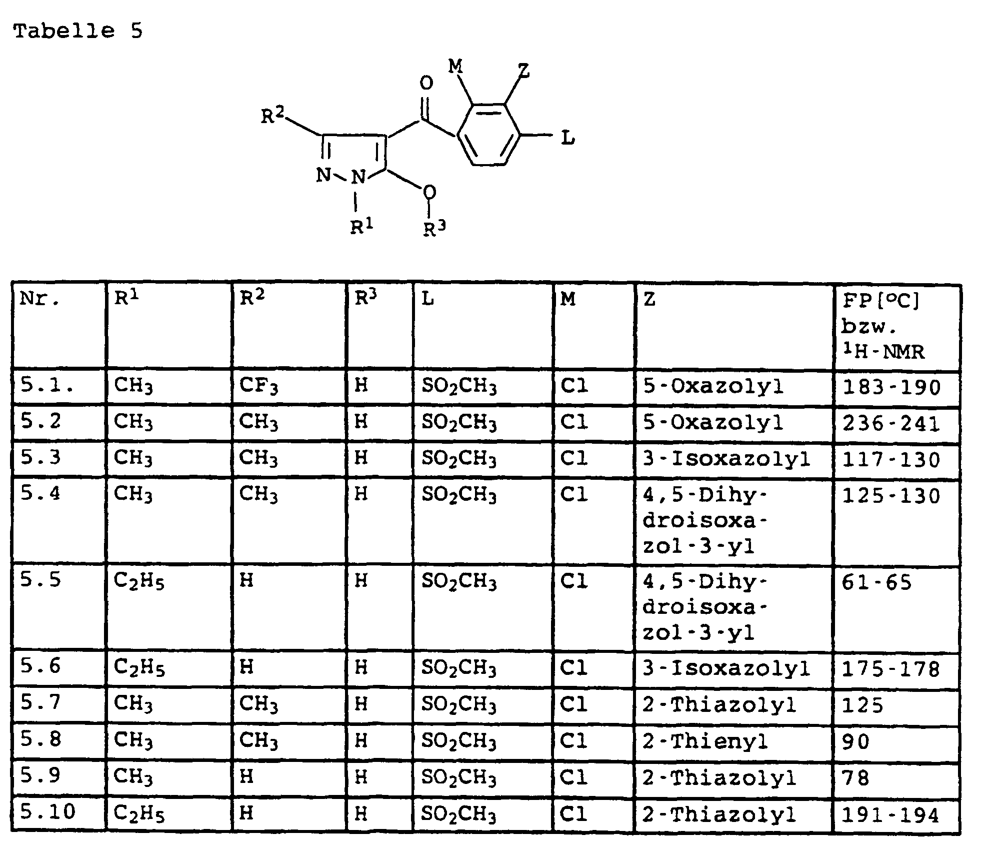

## Patentansprüche

1. Pyrazol-4-yl-benzoylderivate der Formel I 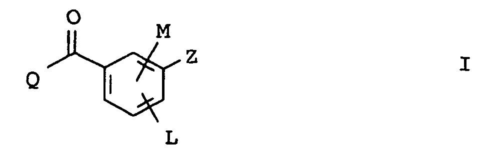 in der die Substituenten folgende Bedeutung haben:
L,M Wasserstoff, C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können, Halogen, Cyano, Nitro, eine Gruppe -(Y)ₙ-S(O)ₘR⁷ oder eine Gruppe -(Y)ₙ-CO-R⁸
Z ein 5- oder 6-gliedriger heterocyclischer, gesättigter oder ungesättigter Rest, enthaltend ein bis drei Heteroatome, ausgewählt aus der Gruppe Sauerstoff, Schwefel oder Stickstoff, der gegebenenfalls durch Halogen, Cyano, Nitro, eine Gruppe -CO-R⁸, C₁-C₄-Alkyl, C₁-C₄-Halogenalkyl, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, gegebenenfalls durch Halogen, Cyano, Nitro, C₁-C₄-Alkyl oder C₁-C₄-Halogenalkyl substituiertes Phenyl oder eine Oxogruppe, die gegebenenfalls auch in der tautomeren Form als Hydroxygruppe vorliegen kann, substituiert ist oder der mit einem ankondensierten, gegebenenfalls durch Halogen, Cyano, Nitro, C₁-C₄-Alkyl oder C₁-C₄-Halogenalkyl substituierten Phenylring, einem ankondensierten Carbocyclus oder einem ankondensierten, gegebenenfalls durch Halogen, Cyano, Nitro, C₁-C₄-Alkyl, Di-C₁-C₄-Alkylamino, C₁-C₄-Alkoxy, C₁-C₄-Halogenalkoxy, oder C₁-C₄-Halogenalkyl substituierten zweiten Heterocyclus ein bicyclisches System bildet.
Y O, NR⁹
n null oder eins
m null, eins oder zwei
R⁷ C₁-C₄-Alkyl, C₁-C₄-Halogenalkyl oder NR⁹R¹⁰
R⁸ C₁-C₄-Alkyl, C₁-C₄-Halogenalkyl, C₁-C₄-Alkoxy, oder NR⁹R¹⁰
R⁹ Wasserstoff oder C₁-C₄-Alkyl
R¹⁰ C₁-C₄-Alkyl
Q ein in Stellung 4 verknüpfter Pyrazolring der Formel II 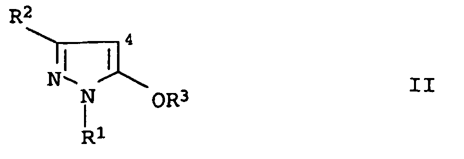
in welcher
R¹ C₁-C₄-Alkyl
R² Wasserstoff, C₁-C₄-Alkyl oder C₁-C₄-Halogenalkyl und
R³ Wasserstoff, C₁-C₄-Alkylsulfonyl, Phenylsulfonyl oder Alkylphenylsulfonyl bedeuten,
sowie landwirtschaftlich übliche Salze der Verbindungen I.

2. Pyrazol-4-yl-benzoylderivate der Formel I, gemäß Anspruch 1, wobei die Reste L und M wie in der Formel Ia angegeben verknüpft sind 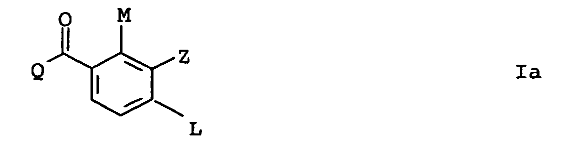 und in der L für C₁-C₆-Alkyl, C₂-C₆-Alkenyl, C₂-C₆-Alkinyl, C₁-C₄-Alkoxy, C₁-C₄-Alkylthio, C₁-C₄-Halogenalkyl, C₁-C₄-Halogenalkoxy, C₁-C₄-Halogenalkylthio, C₁-C₄-Alkylsulfonyl, Halogen, Nitro oder Cyano und M für Wasserstoff, C₁-C₆-Alkyl, C₂-C₆-Alkenyl, C₂-C₆-Alkinyl, C₁-C₄-Alkoxy, C₁-C₄-Alkylthio, C₁-C₄-Halogenalkyl, C₁-C₄-Halogenalkoxy, C₁-C₄-Halogenalkylthio, C₁-C₄-Alkylsulfonyl, Halogen, Nitro oder Cyano steht und Q und Z die in Anspruch 1 angegebenen Bedeutungen haben.

3. Pyrazol-4-yl-benzoylderivate der Formel I, gemäß Anspruch 1, wobei die Reste L und M wie in der Formel Ib angegeben verknüpft sind 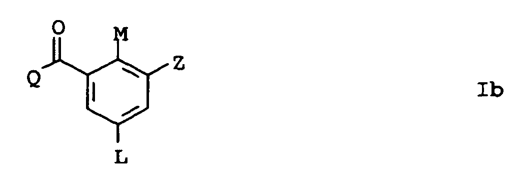 und in der L und M für C₁-C₆-Alkyl, C₂-C₆-Alkenyl, C₂-C₆-Alkinyl, C₁-C₄-Alkoxy, C₁-C₄-Alkylthio, C₁-C₄-Halogenalkyl, C₁-C₄-Halogenalkoxy, C₁-C₄-Halogenalkylthio, C₁-C₄-Alkylsulfonyl, Halogen, Nitro oder Cyano stehen und Q und Z die in Anspruch 1 angegebenen Bedeutungen haben.

4. Pyrazol-4-yl-benzoylderivate der Formel I gemäß Anspruch 1 in der die Reste L bzw. M für Wasserstoff, Methyl, Methoxy, Methylthio, Chlor, Cyano, Methylsulfonyl, Nitro oder Trifluormethyl stehen.

5. Verfahren zur Herstellung der Pyrazol-4-yl-benzoylderivate der Formel I gemäß Anspruch 1, **dadurch gekennzeichnet, daß** man die Pyrazole der Formel IIa 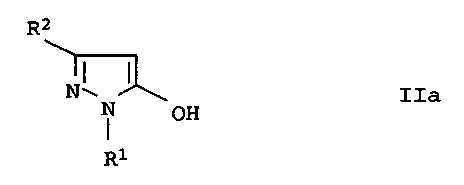 mit einem Benzoylderivat der Formel III 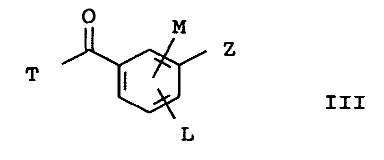 wobei T = Halogen bedeutet und L, M und Z die in Anspruch 1 genannte Bedeutung haben, umsetzt.

6. Herbizides Mittel, enthaltend mindestens ein Pyrazol-4-yl-benzoylderivat der Formel I gemäß Anspruch 1 und übliche inerte Zusatzstoffe.

7. Verfahren zur Bekämpfung unerwünschten Pflanzenwuchses, **dadurch gekennzeichnet, daß** man eine herbizid wirksame Menge eines Pyrazol-4-yl-benzoylderivates der Formel I gemäß Anspruch 1 auf die Pflanzen oder deren Lebensraum einwirken läßt.

8. Pyrazol-4-yl-benzoylderivate der Formel I gemäß Anspruch 1, in der Z ein 5- oder 6-gliedriger Heteroaromat bedeutet, enthaltend ein bis drei Heteroatome, ausgewählt aus der Gruppe Sauerstoff, Schwefel oder Stickstoff, der gegebenenfalls durch Halogen, Cyano, Nitro, C₁-C₄-Alkyl, C₁-C₄-Halogenalkyl, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, gegebenenfalls durch Halogen, Cyano, Nitro, C₁-C₄-Alkyl oder C₁-C₄-Halogenalkyl substituiertes Phenyl substituiert ist oder ein gegebenenfalls durch Halogen, Cyano, Nitro, C₁-C₄-Alkyl oder C₁-C₄-Halogenalkyl substituierter benzokondensierter 5- oder 6-Ring-Heteroaromat;
und L, M und Q die in Anspruch 1 genannte Bedeutung haben.

## Claims

1. A pyrazol-4-ylbenzoyl derivative of the formula I 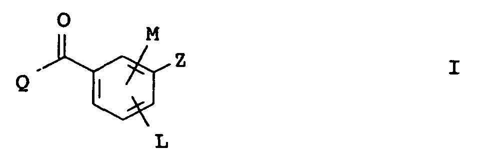 where the substituents are as follows:
L,M are hydrogen, C₁-C₆-alkyl, C₂-C₆-alkenyl, C₂-C₆-alkynyl, C₁-C₄-alkoxy, it being possible for these groups to be unsubstituted or substituted by one to five halogen atoms or C₁-C₄-alkoxy or are halogen, cyano, nitro, a group -(Y)ₙ-S(O)ₘR⁷ or a group -(Y)ₙ-CO-R⁸
Z is a 5- or 6-membered heterocyclic saturated or unsaturated radical which has one to three hetero atoms selected from the group consisting of oxygen, sulfur and nitrogen and which is unsubstituted or substituted by halogen, cyano, nitro, a group -CO-R⁸, C₁-C₄-alkyl, C₁-C₄-haloalkyl, C₃-C₈-cycloalkyl, C₁-C₄-alkoxy, C₁-C₄-haloalkoxy, C₁-C₄-alkylthio, C₁-C₄-haloalkylthio, di-C₁-C₄-alkylamino, by phenyl which is unsubstituted or substituted by halogen, cyano, nitro, C₁-C₄-alkyl or C₁-C₄-haloalkyl, or by an oxo group which may also be present as a hydroxyl group in the tautomeric form, or which forms a bicyclic system together with a fused phenyl ring which is unsubstituted or substituted by halogen, cyano, nitro, C₁-C₄-alkyl or C₁-C₄-haloalkyl or with a fused carbocycle or with a fused second heterocycle which is unsubstituted or substituted by halogen, cyano, nitro, C₁-C₄-alkyl, di-C₁-C₄-alkylamino, C₁-C₄-alkoxy, C₁-C₄-haloalkoxy or C₁-C₄-haloalkyl,
Y is O or NR⁹,
n is zero or 1,
m is zero, 1 or 2,
R⁷ is C₁-C₄-alkyl, C₁-C₄-haloalkyl or NR⁹R¹⁰
R⁸ is C₁-C₄-alkyl, C₁-C₄-haloalkyl, C₁-C₄-alkoxy or NR⁹R¹⁰
R⁹ is hydrogen or C₁-C₄-alkyl,
R¹⁰ is C₁-C₄-alkyl
Q is a pyrazole ring of the formula II 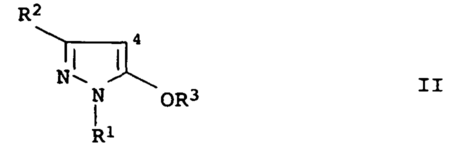
where
R¹ is C₁-C₄-alkyl,
R² is hydrogen, C₁-C₄-alkyl or C₁-C₄-haloalkyl and
R³ is hydrogen, C₁-C₄-alkylsulfonyl, phenylsulfonyl or alkylphenylsulfonyl and
which is linked in the 4-position,
or a salt of a compound I which is customary in agriculture.

2. A pyrazol-4-ylbenzoyl derivative of the formula I as claimed in claim 1 where the radicals L and M are linked as shown in the formula Ia 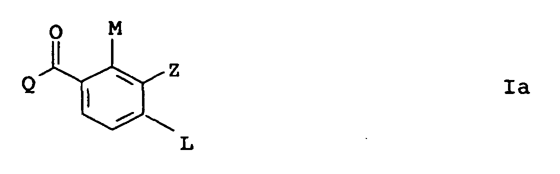 and where L is C₁-C₆-alkyl, C₂-C₆-alkenyl, C₂-C₆-alkynyl, C₁-C₄-alkoxy, C₁-C₄-alkylthio, C₁-C₄-haloalkyl, C₁-C₄-halo-alkoxy, C₁-C₄-haloalkylthio, C₁-C₄-alkylsulfonyl, halogen, nitro or cyano and M is hydrogen, C₁-C₆-alkyl, C₂-C₆-alkenyl, C₂-C₆-alkynyl, C₁-C₄-alkoxy, C₁-C₄-alkylthio, C₁-C₄-haloalkyl, C₁-C₄-haloalkoxy, C₁-C₄-haloalkylthio, C₁-C₄-alkylsulfonyl, halogen, nitro or cyano and Q and Z are as defined in claim 1.

3. A pyrazol-4-ylbenzoyl derivative of the formula I as claimed in claim 1 where the radicals L and M are linked as shown in the formula Ib 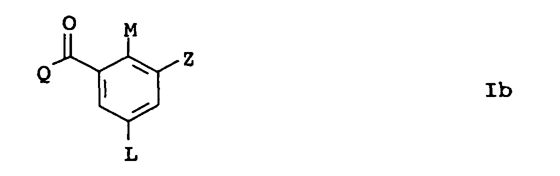 and where L and M are C₁-C₆-alkyl, C₂-C₆-alkenyl, C₂-C₆-alkynyl, C₁-C₄-alkoxy, C₁-C₄-alkylthio, C₁-C₄-haloalkyl, C₁-C₄-haloalkoxy, C₁-C₄-haloalkylthio, C₁-C₄-alkylsulfonyl, halogen, nitro or cyano and Q and Z are as defined in claim 1.

4. A pyrazol-4-ylbenzoyl derivative of the formula I as claimed in claim 1 where the radicals L and M are hydrogen, methyl, methoxy, methylthio, chlorine, cyano, methylsulfonyl, nitro or trifluoromethyl.

5. A process for the preparation of the pyrazol-4-ylbenzoyl derivatives of the formula I as claimed in claim 1, which comprises reacting the pyrazoles of the formula IIa 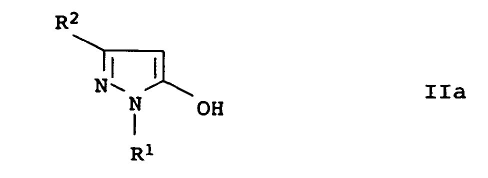 with a benzoyl derivative of the formula III 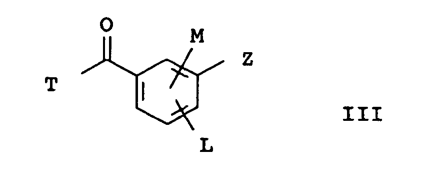 where T is halogen and L, M and Z are as defined in claim 1.

6. A herbicidal composition comprising at least one pyrazol-4-ylbenzoyl derivative of the formula I as claimed in claim 1 and customary inert additives.

7. A method of controlling undesirable vegetation, which comprises allowing a herbicidally active amount of a pyrazol-4-ylbenzoyl derivative of the formula I as claimed in claim 1 to act on the plants or their environment.

8. A pyrazol-4-ylbenzoyl derivative of the formula I as claimed in claim 1 where Z is a 5- or 6-membered heteroaromatic radical which has one to three hetero atoms selected from the group consisting of oxygen, sulfur and nitrogen and which is unsubstituted or substituted by halogen, cyano, nitro, C₁-C₄-alkyl, C₁-C₄-haloalkyl, C₃-C₈-cycloalkyl, C₁-C₄-alkoxy, C₁-C₄-haloalkoxy, C₁-C₄-alkylthio, C₁-C₄-haloalkylthio, di-C₁-C₄-alkylamino, phenyl which is unsubstituted or substituted by halogen, cyano, nitro, C₁-C₄-alkyl or C₁-C₄-haloalkyl, or is a 5- or 6-membered heteroaromatic ring which is benzo-fused and unsubstituted or substituted by halogen, cyano, nitro, C₁-C₄-alkyl or C₁-C₄-haloalkyl,
and L, M and Q are as defined in claim 1.

## Revendications

1. Dérivés de pyrazol-4-yl-benzoyle de la formule I 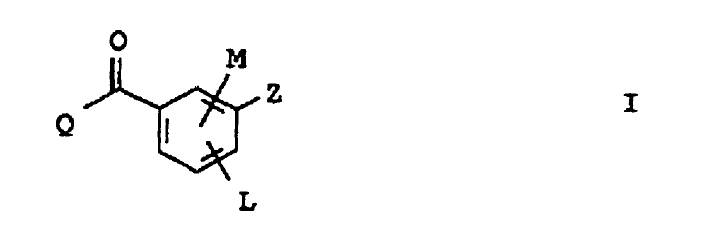 dans laquelle les substituants ont la signification suivante :
L, M représentent de l'hydrogène ou un groupe alkyle en C₁-C₆, alcényle en C₂-C₆, alcynyle en C₂-C₆, alcoxy en C₁-C₄, ces groupes pouvant être éventuellement substitués par 1 à 5 atomes d'halogène ou alcoxy en C₁-C₄, de l'halogène, un groupe cyano, nitro, -(Y)ₙ-S(O)ₘR⁷ ou -(Y)ₙ-CO-R⁸,
Z représente un radical hétérocyclique pentagonal ou hexagonal, saturé ou insaturé, contenant 1 à 3 hétéroatomes choisis parmi le groupe de l'oxygène, du soufre ou de l'azote qui est éventuellement substitué par de l'halogène, un groupe cyano, nitro, -CO-R⁸, alkyle en C₁-C₄, halogénoalkyle en C₁-C₄, cycloalkyle en C₃-C₈, alcoxy en C₁-C₄, halogénoalcoxy en C₁-C₄, alkylthio en C₁-C₄, halogénoalkylthio en C₁-C₄, di-C₁-C₄-alkylamino, phényle, éventuellement substitué par de l'halogène ou un groupe cyano, nitro, alkyle en C₁-C₄ ou halogénoalkyle en C₁-C₄, ou oxo, qui éventuellement peut aussi se présenter dans la forme tautomère comme groupe hydroxy, ou qui forme un système bicyclique avec un noyau de phényle condensé, éventuellement substitué par de l'halogène ou un groupe cyano, nitro, alkyle en C₁-C₄ ou halogénoalkyle en C₁-C₄, un carbocycle condensé ou un deuxième hétérocycle condensé, éventuellement substitué par de l'halogène ou un groupe cyano, nitro, alkyle en C₁-C₄, di-C₁-C₄-alkylamino, alcoxy en C₁-C₄, halogénoalcoxy en C₁-C₄ ou halogénoalkyle en C₁-C₄,
Y représente O, NR⁹,
n vaut 0 ou 1,
m vaut 0, 1 ou 2,
R⁷ représente un groupe alkyle en C₁-C₄, halogénoalkyle en C₁-C₄ ou NR⁹R¹⁰,
R⁸ représente un groupe alkyle en C₁-C₄, halogénoalkyle en C₁-C₄, alcoxy en C₁-C₄ ou NR⁹R¹⁰,
R⁹ représente de l'hydrogène ou un groupe alkyle en C₁-C₄,
R¹⁰ représente un groupe alkyle en C₁-C₄,
Q représente un noyau de pyrazole enchaîné en position 4 de la formule II 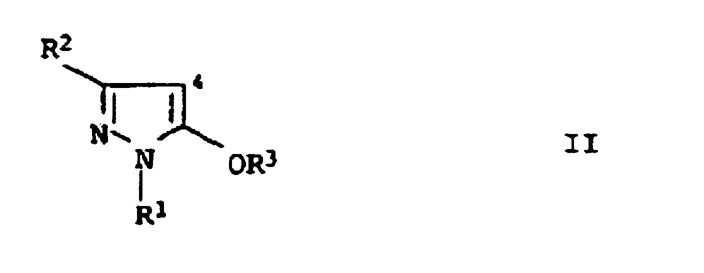
dans laquelle
R¹ représente un groupe alkyle en C₁-C₄,
R² représente de l'hydrogène ou un groupe alkyle en C₁-C₄, ou halogénoalkyle en C₁-C₄, et
R³ représente de l'hydrogène ou un groupe alkylsulfonyle en C₁-C₄, phénylsulfonyle ou alkylphénylsulfonyle,
ainsi que les sels courants en agriculture des composés I.

2. Dérivés de pyrazol-4-yl-benzoyle de la formule I suivant la revendication 1, dans laquelle les radicaux L et M sont enchaînés comme indiqué dans la formule Ia 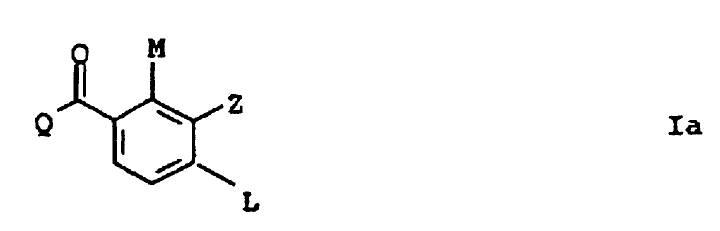 où L représente un groupe alkyle en C₁-C₆, alcényle en C₂-C₆, alcynyle en C₂-C₆, alcoxy en C₁-C₄, alkylthio en C₁-C₄, halogénoalkyle en C₁-C₄, halogénoalcoxy en C₁-C₄, halogénoalkylthio en C₁-C₄, alkylsulfonyle en C₁-C₄, nitro ou cyano ou de l'halogène et M représente de l'hydrogène, de l'halogène ou un groupe alkyle en C₁-C₆, alcényle en C₂-C₆, alcynyle en C₂-C₆, alcoxy en C₁-C₄, alkylthio en C₁-C₄, halogénoalkyle en C₁-C₄, halogénoalcoxy en C₁-C₄, halogénoalkylthio en C₁-C₄, alkylsulfonyle en C₁-C₄, nitro ou cyano et Q et Z ont les significations indiquées dans la revendication 1.

3. Dérivés de pyrazol-4-yl-benzoyle de la formule I suivant la revendication 1, dans laquelle les radicaux L et M sont enchaînés comme indiqué dans la formule Ib 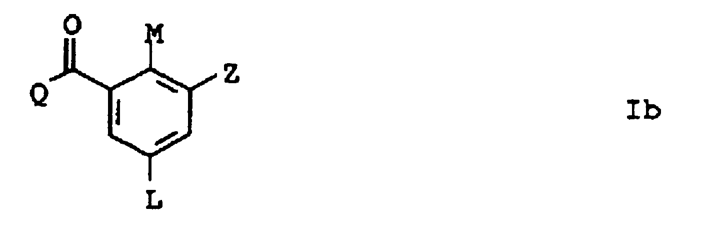 où L et M représentent de l'halogène ou un groupe alkyle en C₁-C₆, alcényle en C₂-C₆, alcynyle en C₂-C₆, alcoxy en C₁-C₄, alkylthio en C₁-C₄, halogénoalkyle en C₁-C₄, halogénoalcoxy en C₁-C₄, halogénoalkythio en C₁-C₄, alkylsulfonyle en C₁-C₄, nitro ou cyano et Q et Z ont les significations indiquées dans la revendication 1.

4. Dérivés de pyrazol-4-yl-benzoyle de la formule I suivant la revendication 1, dans laquelle les radicaux L et respectivement M représentent de l'hydrogène, du méthyle, du méthoxy, du méthylthio, du chlore, du cyano, du méthylsulfonyle, du nitro ou du trifluorométhyle.

5. Procédé de préparation des dérivés de pyrazol-4-yl-benzoyle de la formule I suivant la revendication 1, **caractérisé en ce qu'**on fait réagir les pyrazoles de la formule IIa 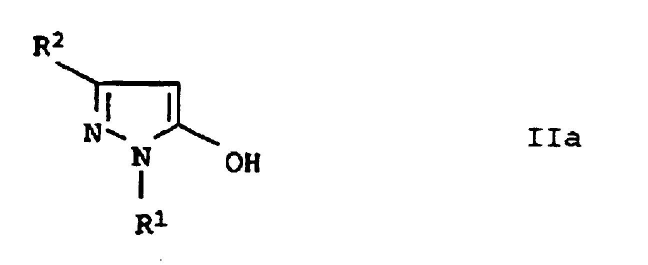 avec un dérivé de benzoyle de la formule III 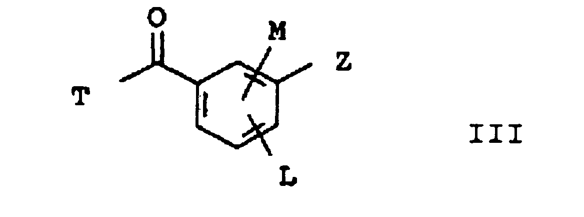 où T représente de l'halogène et L, M et Z ont la signification indiquée dans la revendication 1.

6. Produit herbicide contenant au moins un dérivé de pyrazol-4-yl-benzoyle de la formule I suivant la revendication 1 et des additifs inertes courants.

7. Procédé de lutte contre la croissance non désirée de plantes, **caractérisé en ce qu'**on fait agir sur les plantes ou leur environnement une quantité efficace du point de vue herbicide d'un dérivé de pyrazol-4-yl-benzoyle de la formule I suivant la revendication 1.

8. Dérivés de pyrazol-4-yl-benzoyle de la formule I suivant la revendication 1, dans laquelle Z représente une substance hétéroaromatique pentagonale ou hexagonale, qui contient un à trois hétéroatomes choisis parmi le groupe de l'oxygène, du soufre ou de l'azote et qui est éventuellement substitué par de l'halogène ou un groupe cyano, nitro, alkyle en C₁-C₄, halogénoalkyle en C₁-C₄, cycloalkyle en C₃-C₈, alcoxy en C₁-C₄, halogénoalcoxy en C₁-C₄, alkylthio en C₁-C₄, halogénoalkylthio en C₁-C₄, di-C₁-C₄-alkylamino ou phényle substitué éventuellement par de l'halogène ou un groupe cyano, nitro, alkyle en C₁-C₄ ou halogénoalkyle en C₁-C₄, ou une substance hétéroaromatique à noyau pentagonal ou hexagonal benzocondensée, substituée éventuellement par de l'halogène ou un groupe cyano, nitro, alkyle en C₁-C₄ ou halogénoalkyle en C₁-C₄,
et L, M et Q ont la signification indiquée dans la revendication 1.
